# EUROPEAN PATENT APPLICATION

(11) **EP 4 008 776 A1**
(43) Date of publication of application: **08.06.2022**
(21) Application number: 20848196.0
(22) Date of filing: 08.06.2020
(51) Int. Cl.: C12N 9/10, A23K 20/163, A23L 33/10, A61K 31/702, A61K 31/715, C12N 1/15, C12N 1/19, C12N 1/21, C12N 5/10, C12N 15/54, C12P 19/18

(54) **PROTEIN HAVING ACTIVITY OF CATALYZING ALPHA-1,6-GLUCOSYL TRANSFER REACTION**

(30) Priority: 01.08.2019 JP 2019142406
(71) Applicant: Nihon Shokuhin Kako Co., Ltd., Tokyo 100-0005 (JP)
(72) Inventor: KANAI, Kenta, Fuji-shi, Shizuoka 417-8530 (JP); AIZAWA, Kenta, Fuji-shi, Shizuoka 417-8530 (JP); KANAI, Mioka, Fuji-shi, Shizuoka 417-8530 (JP); TAKECHI, Noriaki, Fuji-shi, Shizuoka 417-8530 (JP); IIZUKA, Takahisa, Fuji-shi, Shizuoka 417-8530 (JP)
(74) Representative: Michalski Hüttermann & Partner Patentanwälte mbB
(86) International application number: PCT/JP2020/022467
(87) International publication number: WO 2021/019912

(57) **Abstract**

An object of the present invention is to provide a protein that catalyzes an α-1,6-glucosyl transfer reaction and can efficiently produce an α-1,6-glucan, an enzyme preparation for producing an α-1,6-glucan, which comprise said protein as an active ingredient, and a method for producing an α-1,6-glucan using the enzyme preparation. The present invention provides a protein having an activity for catalyzing an α-1,6-glucosyl transfer reaction, which is any of the proteins (a) to (c) mentioned below: (a) a protein consisting of the amino acid sequence of SEQ ID NO: 3; (b) a protein consisting of an amino acid sequence having an amino acid sequence identity of 90% or higher to the amino acid sequence of SEQ ID NO: 3; and (c) a protein consisting of an amino acid sequence in which one or several amino acid(s) have been substituted, inserted, deleted and/or added in the amino acid sequence of SEQ ID NO: 3. The present invention provides an enzyme preparation for use in production of an α-1,6-glucan from an oligosaccharide and/or polysaccharide having an α-1,4-glucosidic linkage and/or an α-1,6-glucosidic linkage, which contains the aforementioned protein. The present invention provides a method for producing an α-1,6-glucan, which comprises the reaction step of allowing the aforementioned enzyme preparation to act on an oligosaccharide and/or polysaccharide having an α-1,4-glucosidic linkage and/or an α-1,6-glucosidic linkage to obtain an α-1,6-glucan.

## Description

### Technical Field

The present invention relates to a protein having an activity for catalyzing an a-1,6-glucosyl transfer reaction, and an enzyme preparation containing said protein, which is used for producing an α-1,6-glucan. The present invention relates to a method for producing an α-1,6-glucan comprising the step of allowing the aforementioned enzyme preparation to act on an oligosaccharide and/or polysaccharide having an α-1,4-glucosidic linkage and/or α-1,6-glucosidic linkage to obtain an α-1,6-glucan.

### Cross-reference of related application

This application claims convention priority based on Japanese Patent Application No. 2019-142406 filed in Japan on August 1, 2019, of which entire description is herein incorporated by reference.

### Background Technique

It has been reported that carbohydrates consisting of mainly α-1,6-linked D-glucoses (α-1,6-glucan) are slowly and sustainably digestible (Patent document 1). Slowly digestible and sustainably digestible carbohydrates are useful as carbohydrates that can be used even by diabetic patients who need to avoid a rapid increase in blood glucose levels, because such carbohydrates provide mild increase in blood glucose levels even after ingestion thereof. Among α-1,6-glucans, it has been reported that isomaltomegalosaccharides having a degree of polymerization (DP) of 10 to 50 have an effect of enhancing the intestinal barrier function (Patent document 2). It has also been reported that anchor type isomaltomegalosaccharides having an anchor sugar chain constituted with α-1,4-linkages at both ends or only the nonreducing end of an isomaltomegalosaccharide chain (DP 10 to 100) have an effect of promoting dissolution of hardly water-soluble compounds (Patent document 3). Thus, α-1,6-glucans are expected to be a useful carbohydrate material in various fields such as food and medicine.

As enzymatic production method of α-1,6-glucans, there have been disclosed a method using a dextransucrase derived from *Leuconostoc mesenteroides* (Patent document 4), methods using a dextrin dextranase derived from *Gluconobacter oxydans* (Patent documents 5 and 6), a method using a dextran glucanase derived from the 598K strain belonging to *Paenibacillus* sp. (Patent document 7), and a method using an enzyme having an α-1,6-glucosyl transfer activity derived from *Thermoanaerobacter siderophilus* (Patent document 8).

However, the production method using a dextran sucrase mentioned above has a problem that only the glucose portion of sucrose as the starting material is utilized in this method, and therefore the yield of dextran to sugar never exceeds 50%.

On the other hand, dextrin dextranase, dextran glucanase, and enzymes having an a-1,6-glucosyl transfer activity can produce α-1,6-glucan using a partial degradation product of starch as a substrate. In particular, the enzyme having an α-1,6-glucosyl transfer activity derived from *Thermoanaerobacter siderophilus* is stable up to 60°C, and can be heterologously expressed by using *Bacillus subtilis* as a host (Patent document 8).

### Prior Art References

### Patent documents

Patent document 1: WO2016/047616
Patent document 2: Japanese Patent Unexamined Publication (Kokai) No. 2015-205856
Patent document 3: Japanese Patent Unexamined Publication (Kokai) No. 2017-114943
Patent document 4: Japanese Patent Unexamined Publication (Kokai) No. Hei 8-173178
Patent document 5: Japanese Patent Unexamined Publication (Kokai) No. 2001-258589
Patent document 6: Japanese Patent Unexamined Publication (Kokai) No. 2007-181452
Patent document 7: Japanese Patent Unexamined Publication (Kokai) No. 2012-095606
Patent document 8: Japanese Patent No. 6417061

The entire descriptions of Patent documents 1 to 8 are incorporated herein by reference.

### Summary of the Invention

### Object to be achieved by the invention

Although enzymes having an α-1,6-glucosyl transfer activity have been conventionally known, there has been desired to develop enzymes capable of more efficiently producing α-1,6-glucans. An object of the present invention is to provide a protein having an activity for catalyzing an a-1,6-glucosyl transfer reaction that can efficiently produce an α-1,6-glucan, an enzyme preparation for producing an α-1,6-glucan comprising said protein as an active ingredient, and a method for producing an α-1,6-glucan using said enzyme preparation.

### Means for achieving the object

The inventors of the present invention conducted various studies in order to achieve the aforementioned object, as a result, found a protein that can produce an α-1,6-glucan more efficiently compared with conventional enzymes having an α-1,6-glucosyl transfer activity, and accomplished the present invention. The present invention is based on this finding.

The present invention provides the following inventions.
[1] A protein having an activity for catalyzing an α-1,6-glucosyl transfer reaction, which is any of the proteins (a) to (c) mentioned below:
   (a) a protein consisting of the amino acid sequence of SEQ ID NO: 3:
   (b) a protein consisting of an amino acid sequence having an amino acid sequence identity of 90% or higher to the amino acid sequence of SEQ ID NO: 3; and
   (c) a protein consisting of an amino acid sequence in which one or several amino acid(s) have been substituted, inserted, deleted and/or added in the amino acid sequence of SEQ ID NO: 3.
[2] A protein derived from *Tepidibacillus decaturensis* and having the following properties:
   (1) the protein has an activity for catalyzing an α-1,6-glucosyl transfer reaction;
   (2) the molecular weight measured by SDS-PAGE is from 95,000 to 105,000;
   (3) the optimum pH is 3.9 to 4.6;
   (4) the stable pH range is 4.2 to 9.0;
   (5) the optimum temperature is 50 to 55°C; and
   (6) the temperature stability is maintained at 50°C or lower.
[3] A culture supernatant of a transformed cell into which any of the polynucleotides (a) to (c) mentioned below has been introduced:
   (a) a polynucleotide consisting of the nucleotide sequence of SEQ ID NO: 4;
   (b) a polynucleotide that hybridizes with a complementary strand of the polynucleotide consisting of the nucleotide sequence of SEQ ID NO: 4 under stringent conditions; and
   (c) a polynucleotide having a sequence identity of 90% or higher to the polynucleotide consisting of the nucleotide sequence of SEQ ID NO: 4
[4] An enzyme preparation for use in production of an α-1,6-glucan from an oligosaccharide and/or polysaccharide having an α-1,4-glucosidic linkage and/or an α-1,6-glucosidic linkage, which contains the protein according to [1] or [2] and/or the culture supernatant according to [3].
[5] The enzyme preparation according to [4], wherein the oligosaccharide and/or polysaccharide having an α-1,4-glucosidic linkage and/or an α-1,6-glucosidic linkage is a starch partial degradation product.
[6] The enzyme preparation according to [4] or [5], wherein the α-1,6-glucan is an isomaltooligosaccharide and/or isomaltomegalosaccharide having a degree of polymerization of from 2 to 30.
[7] A composition containing any of the proteins (a) to (c) mentioned below and having an activity for catalyzing an a-1,6-glucosyl transfer reaction:
   (a) a protein consisting of the amino acid sequence of SEQ ID NO: 3;
   (b) a protein consisting of an amino acid sequence having an amino acid sequence identity of 90% or higher to the amino acid sequence of SEQ ID NO: 3; and
   (c) a protein consisting of an amino acid sequence in which one or several amino acid(s) have been substituted, inserted, deleted and/or added in the amino acid sequence of SEQ ID NO: 3.
[8] The composition according to [7], which is for use in production of an α-1,6-glucan from an oligosaccharide and/or polysaccharide having an α-1,4-glucosidic linkage and/or an α-1,6-glucosidic linkage.
[9] A method for producing an α-1,6-glucan, which comprises the reaction step of allowing the enzyme preparation according to any one of [4] to [6] or the composition according to [7] or [8] to act on an oligosaccharide and/or polysaccharide having an α-1,4-glucosidic linkage and/or an α-1,6-glucosidic linkage to obtain an α-1,6-glucan.
[10] The production method according to [9], which comprises the following step to be performed prior to the aforementioned reaction step:
   the step of hydrolyzing starch to obtain an oligosaccharide and/or polysaccharide having an α-1,4-glucosidic linkage and/or an α-1,6-glucosidic linkage.
[11] A method for producing a food, feed, feed for fish culture, cosmetic, or medicament, which comprises the step of performing the method according to [9] or [10] to obtain an α-1,6-glucan, and the step of obtaining a food, feed, feed for fish culture, cosmetic, or medicament by using the α-1,6-glucan obtained in the foregoing step.
[12] A method for producing a glycoside, which comprises the step of allowing the enzyme preparation according to any one of [4] to [6] or the composition according to [7] or [8] to act on a sugar acceptor and a sugar donor.
[13] The method for producing a glycoside according to [12], wherein the sugar donor is a maltooligosaccharide.
[14] The method for producing a glycoside according to [12] or [13], wherein the sugar acceptor is a compound having an alcoholic hydroxyl group or a compound having a phenolic hydroxyl group.
[15] A method for producing a food, feed, feed for fish culture, cosmetic, or medicament, which comprises the step of performing the method according to any one of [12] to [14] to obtain a glycoside, and the step of obtaining a food, feed, feed for fish culture, cosmetic, or medicament by using the glycoside obtained in the foregoing step.

### Effect of the Invention

According to the present invention, a novel protein having an activity for catalyzing an a-1,6-glucosyl transfer reaction can be provided. According to the present invention, an α-1,6-glucan can be more efficiently produced compared with use of conventional enzymes having an activity for catalyzing an a-1,6-glucosyl transfer reaction. According to the present invention, an α-1,6-glucan can be produced by using starch hydrolysate (starch partial degradation product) as a starting material (substrate).

### Brief Description of the Drawings

[Fig. 1A] Fig. 1A shows an amino acid sequence of a hypothetical protein derived from *Tepidibacillus decaturensis* (SEQ ID NO: 1). The underlined region is a signal peptide sequence.
[Fig. 1B] Fig. 1B shows a nucleotide sequence encoding the amino acid sequence of the hypothetical protein derived from *Tepidibacillus decaturensis* (SEQ ID NO: 2). The underlined region is a nucleotide sequence corresponding to the signal peptide sequence.
[Fig. 2] Fig. 2 shows results of SDS-PAGE of the hypothetical protein derived from *Tepidibacillus decaturensis* secreted by *Bacillus subtilis* and purified. M, molecular weight marker; and S, purified protein (C-terminal His-tag).
[Fig. 3] Fig. 3 shows results of SDS-PAGE of a culture supernatant obtained by secretion of the hypothetical protein derived from *Tepidibacillus decaturensis* by *Bacillus subtilis.* The band of the hypothetical protein is indicated with an arrow. M, molecular weight marker; and S, culture supernatant.

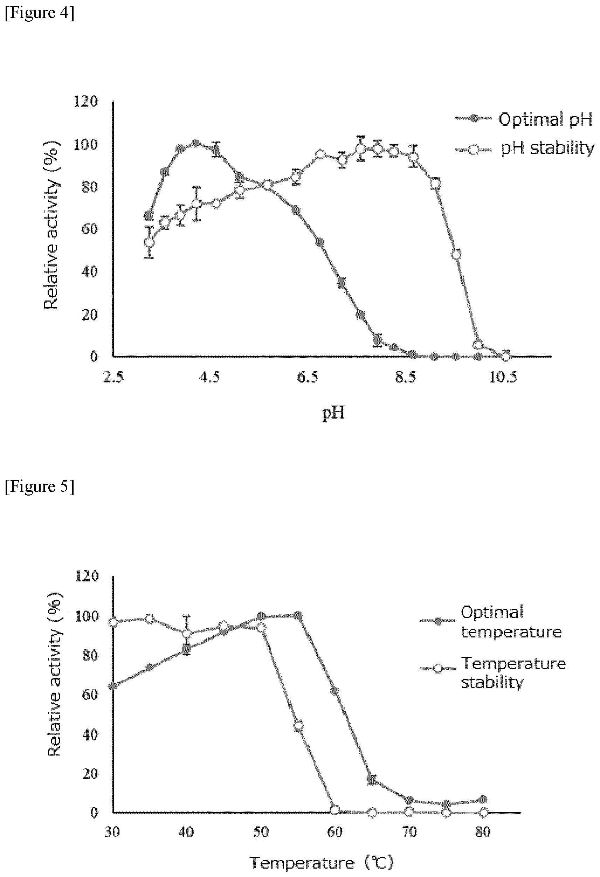

[Fig. 4] Fig. 4 is a graph showing the relative enzyme activities of the purified protein at different pH values. The black circles (•) indicate the relative activities (%) at various pH values based on the maximum enzyme activity (pH 4.2), which is taken as 100%, and the white circles (○) indicate the residual activities (%) observed after 24 hours of retention at 4°C in buffers of various pH values (pH 2.5 to 11.0, 0.5 increments). The pH values listed in the graph are the actually measured values in the reaction solutions for the optimum pH, and the values actually measured in the buffers after adding the protein and retaining the buffers for pH stability.
[Fig. 5] Fig. 5 is a graph showing the relative enzyme activities of the purified protein at temperatures of from 30 to 80°C. The black circles (•) indicate the relative activities (%) at various temperatures based on the maximum enzyme activity (temperature 55°C), which is taken as 100%, and the white circles (○) indicate the residual activities (%) observed after retention at various temperatures for 60 minutes.
[Fig. 6] Fig. 6 is a chromatogram obtained in HPLC analysis of G67 rich syrup. The peaks at the retention times of 74.810 and 71.327 minutes indicate DP6 and 7 carbohydrates, respectively.

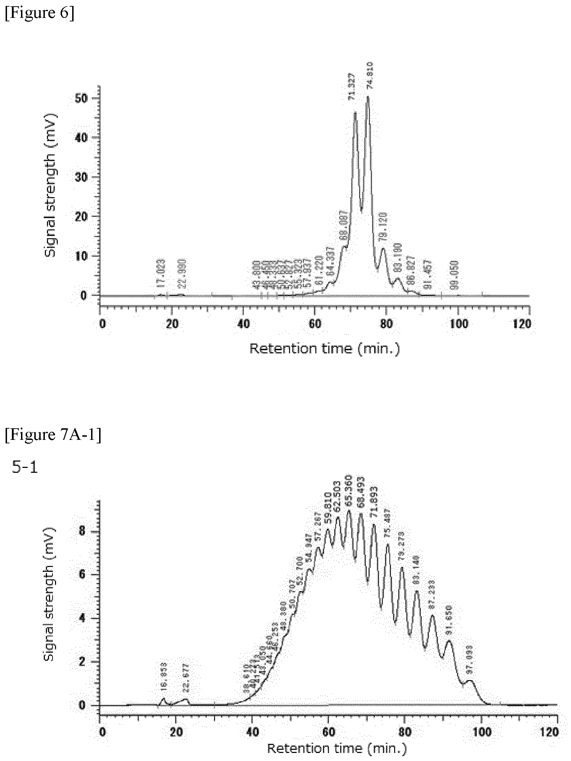

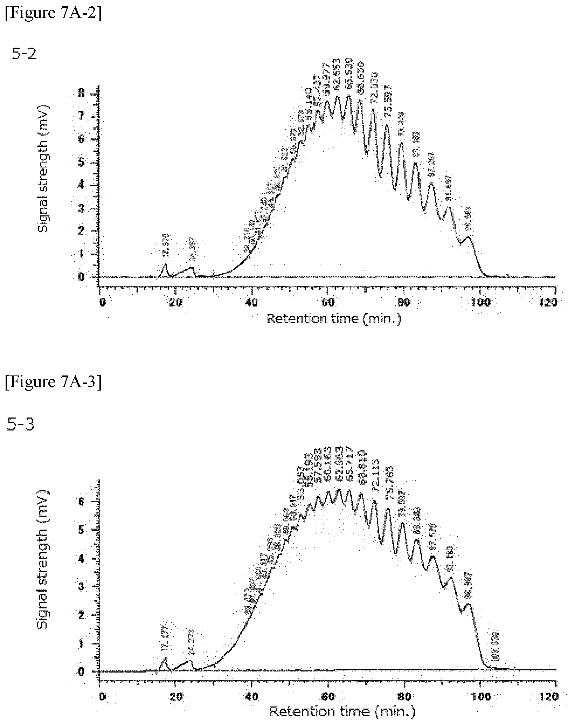

[Fig. 7A-1] Figs 7A-1 to 7A-3 show the results of HPLC analysis of the reaction products produced from the G67-rich syrup as the starting material. The reaction products 5-1 to 5-3 were obtained with different addition amounts of the protein, and the addition amounts of the protein were 31.2, 62.4, and 125 µL/g-ds, respectively. In the chromatograms of the reaction products 5-1 to 5-3, the peaks locating at a retention time of 96 to 98 minutes indicate DP1 carbohydrates, and the following peaks from the next peak on the left side (shorter retention times) indicate DP2 and higher carbohydrates with the numbers increasing in that order.
[FIG. 7A-2] Figs 7A-1 to 7A-3 show the results of HPLC analysis of the reaction products produced from the G67-rich syrup as the starting material. The reaction products 5-1 to 5-3 were obtained with different addition amounts of the protein, and the addition amounts of the protein were 31.2, 62.4, and 125 µL/g-ds, respectively. In the chromatograms of the reaction products 5-1 to 5-3, the peaks locating at a retention time of 96 to 98 minutes indicate DP1 carbohydrates, and the following peaks from the next peak on the left side (shorter retention times) indicate DP2 and higher carbohydrates with the numbers increasing in that order.
[Fig. 7A-3] Figs 7A-1 to 7A-3 show the results of HPLC analysis of the reaction products produced from the G67-rich syrup as the starting material. The reaction products 5-1 to 5-3 were obtained with different addition amounts of the protein, and the addition amounts of the protein were 31.2, 62.4, and 125 µL/g-ds, respectively. In the chromatograms of the reaction products 5-1 to 5-3, the peaks locating at a retention time of 96 to 98 minutes indicate DP1 carbohydrates, and the following peaks from the next peak on the left side (shorter retention times) indicate DP2 and higher carbohydrates with the numbers increasing in that order.

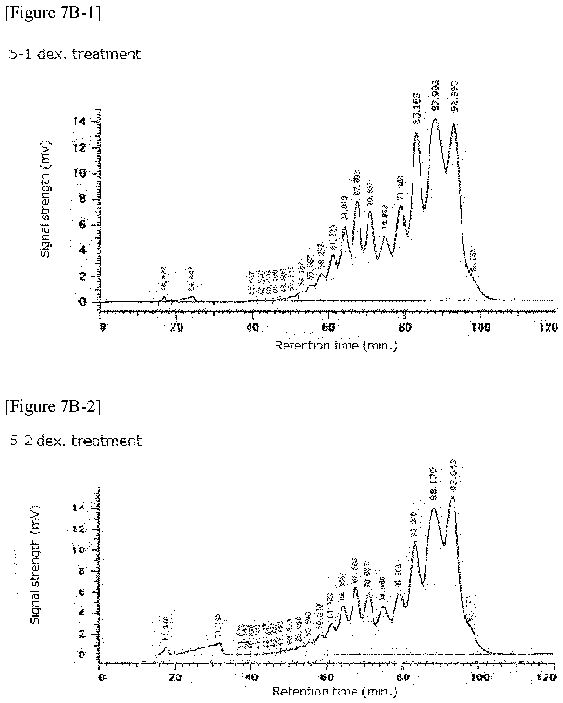

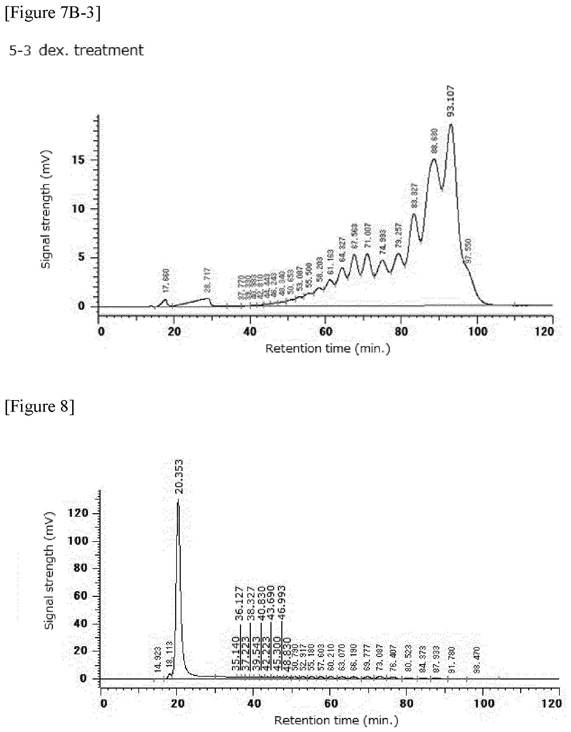

[Fig. 7B-1] Figs. 7B-1 to 7B-3 show the chromatograms of the reaction products 5-1 to 5-3 obtained after dextranase (dex.) treatment. In the chromatograms of the reaction products 5-1 to 5-3 obtained after dextranase (dex.) treatment, the peaks locating at a retention time of 97 to 99 minutes show DP1 carbohydrates, and the following peaks from the next peak on the left side (shorter retention times) indicate DP2 and higher carbohydrates with the numbers increasing in that order.
[Fig. 7B-2] Figs. 7B-1 to 7B-3 show the chromatograms of the reaction products 5-1 to 5-3 obtained after dextranase (dex.) treatment. In the chromatograms of the reaction products 5-1 to 5-3 obtained after dextranase (dex.) treatment, the peaks locating at a retention time of 97 to 99 minutes show DP1 carbohydrates, and the following peaks from the next peak on the left side (shorter retention times) indicate DP2 and higher carbohydrates with the numbers increasing in that order.
[Fig. 7B-3] Figs. 7B-1 to 7B-3 show the chromatograms of the reaction products 5-1 to 5-3 obtained after dextranase (dex.) treatment. In the chromatograms of the reaction products 5-1 to 5-3 obtained after dextranase (dex.) treatment, the peaks locating at a retention time of 97 to 99 minutes show DP1 carbohydrates, and the following peaks from the next peak on the left side (shorter retention times) indicate DP2 and higher carbohydrates with the numbers increasing in that order.
[Fig. 8] Fig. 8 shows the chromatogram obtained by HPLC analysis of the starch partial degradation product (liquefying liquid) used as a starting material.

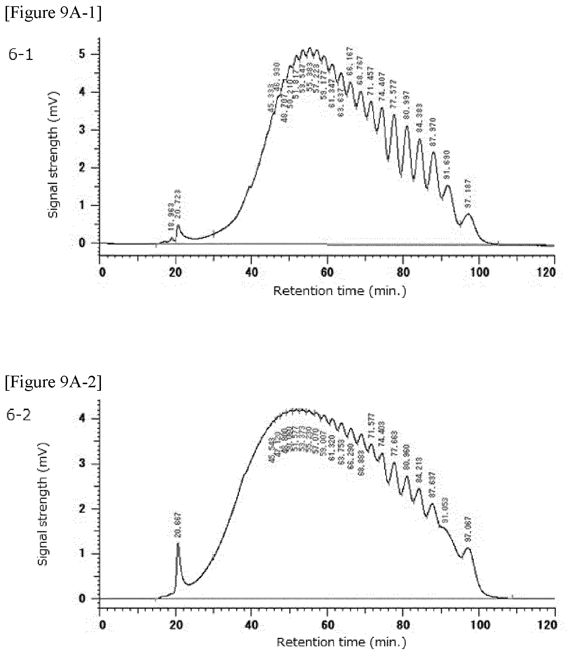

[Fig. 9A-1] Figs. 9A-1 and 9A-2 show chromatograms obtained by HPLC analysis of the reaction products produced from a starch partial degradation product (liquefying liquid). The reaction products 6-1 and 6-2 were obtained with different addition amounts of the protein, and addition amounts of the protein were 31.2 and 125 µL/g-ds, respectively. The peaks locating at a retention time of about 97 minutes indicate DP1 carbohydrates, and the following peaks from the next peak on the left side (shorter retention times) indicate DP2 and higher carbohydrates with the numbers increasing in that order.
[Fig. 9A-2] Figs. 9A-1 and 9A-2 show chromatograms obtained by HPLC analysis of the reaction products produced from a starch partial degradation product (liquefying liquid). The reaction products 6-1 and 6-2 were obtained with different addition amounts of the protein, and addition amounts of the protein were 31.2 and 125 µL/g-ds, respectively. The peaks locating at a retention time of about 97 minutes indicate DP1 carbohydrates, and the following peaks from the next peak on the left side (shorter retention times) indicate DP2 and higher carbohydrates with the numbers increasing in that order.

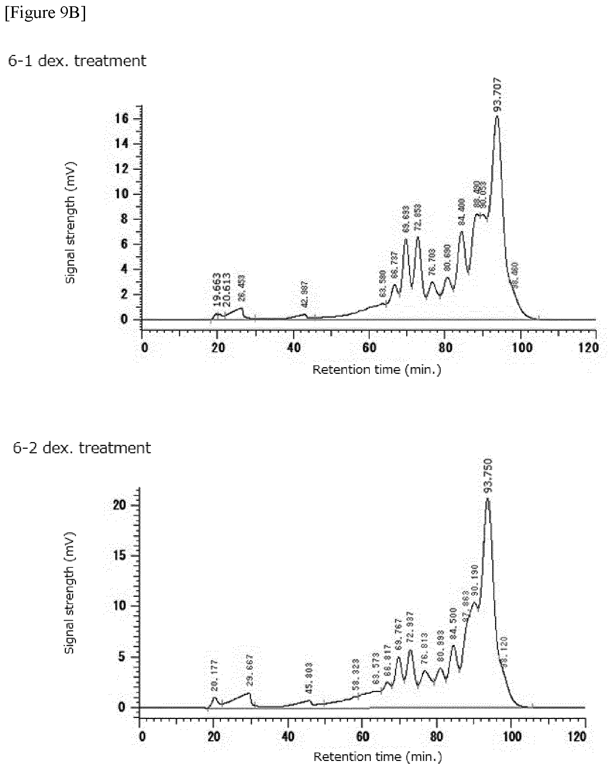

[Fig. 9B] Fig. 9B shows the chromatograms of the reaction products 6-1 and 6-2 subjected to dextranase (dex.) treatment. The peaks locating at a retention time of about 98 minutes indicate DP1 carbohydrates, and the following peaks from the next peak on the left side (shorter retention times) indicate DP2 and higher carbohydrates with the numbers increasing in that order.

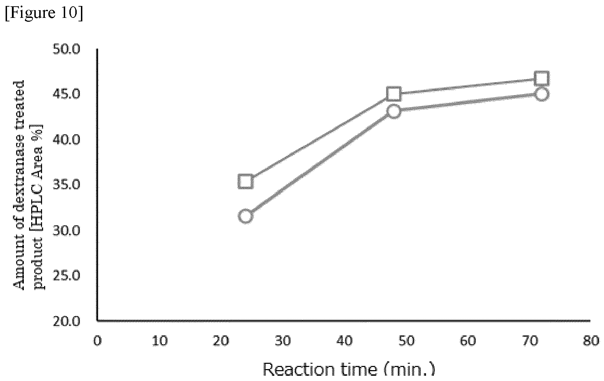

[Fig. 10] Fig. 10 is a graph showing the change of the amount of dextranase treated product (HPLC Area %) over time. The white circles (○) and white squares (□) indicate the increases in DP1 to 3 observed after the dextranase treatment for the reaction product 6-1 and the reaction product 6-2, respectively.

### Modes for Carrying out the Invention

The explanations of the present invention described below may be made with reference to representative embodiments or specific examples thereof, but the present invention is not limited to such embodiments. In this description, numerical ranges expressed by using "to" means a range including the numerical values described before and after "to" as the minimum and maximum values. The degree of polymerization (DP) described in connection with the present invention means a degree of polymerization of glucose as the constituent sugar, regardless of the type of the glucosidic linkage. As for the sugar composition (%) of the reaction product produced by the protein of the present invention, the ratio of each sugar was calculated as the area ratio (%) of the peak corresponding to each sugar to the total area of the peaks detected by HPLC, which was taken as 100.

### (Protein)

The present invention provides a protein having an activity for catalyzing an a-1,6-glucosyl transfer reaction, which is any of the proteins (a) to (c) mentioned below:
(a) a protein consisting of the amino acid sequence of SEQ ID NO: 3;
(b) a protein consisting of an amino acid sequence having an amino acid sequence identity of 90% or higher to the amino acid sequence of SEQ ID NO: 3; and
(c) a protein consisting of an amino acid sequence in which one or several amino acid(s) have been substituted, inserted, deleted and/or added in the amino acid sequence of SEQ ID NO: 3.

The protein consisting of the amino acid of SEQ ID NO: 3 has a sequence corresponds to the amino acid sequence of SEQ ID NO: 1 obtained from the genomic information of the microaerophilic bacterium, *Tepidibacillus decaturensis* (NCBI Reference Sequence: WP_068722961.1), of which signal sequence is deleted. The annotation of the amino acid sequence of SEQ ID NO: 1 is a hypothetical protein, and the specific properties of the activity thereof have not been known prior to this application.

The inventors of the present invention found that the protein consisting of the amino acid sequence of SEQ ID NO: 3 has an activity for catalyzing an α-1,6-glucosyl transfer reaction. In this description, "activity for catalyzing an α-1,6-glucosyl transfer reaction" or "α-1,6-glucosyl transfer activity" refers to an activity for catalyzing a reaction of forming an α-1,6 -glucosidic linkage through a sugar transfer reaction.

The a-1,6-glucosyl transfer activity can be evaluated by reacting a protein having an a-1,6-glucosyl transfer activity with any of maltose, maltotriose, isomaltose, and isomaltotriose as a substrate, and detecting saccharides extended with glucoses transferred from the substrate with α-1,6 linkages in a resulting reaction product.

Specifically, the α-1,6-glucosyl transfer activity can be evaluated by, for example, adding a protein to be evaluated for the a-1,6-glucosyl transfer activity to a reaction solution containing a high maltose content syrup to perform an enzymatic treatment at 53°C for 72 hours, and analyzing the reaction mixture obtained after the enzymatic treatment by high performance liquid chromatography (HPLC) to detect sugars transferred and used for extension through α-1,6 linkages. It can be confirmed that the transfer of the sugars and extension are attained with α-1,6 linkages by further treating the reaction mixture obtained after the enzymatic treatment with a dextranase and detecting the degraded sugars by HPLC analysis.

The protein of the present invention may also be a protein consisting of an amino acid sequence having an amino acid sequence identity of 90% or higher, 91% or higher, 92% or higher, 93% or higher, 94% or higher, 95% or higher, 96% or higher, 97% or higher, 98% or higher, or 99% or higher to the amino acid sequence of SEQ ID NO: 3 and exhibiting an α-1,6-glucosyl transfer activity. The amino acid sequence identity is defined as the percentage of amino acid residues that are identical between two aligned amino acid sequences to be compared, wherein gaps are introduced in order to obtain the maximum sequence identity, if necessary. The amino acid sequence identity can be determined by using publicly available computer software such as, for example, BLAST, BLAST-2, ALIGN, and Megalign (DNASTAR) software.

Further, the protein of the present invention may also be a protein consisting of an amino acid sequence in which one or several amino acid(s) have been substituted, inserted, deleted and/or added in the amino acid sequence of SEQ ID NO: 3 and having an a-1,6-glucosyl transfer activity. The range of "one to several" in the expression of "an amino acid sequence derived by substitution, insertion, deletion and/or addition of one or several amino acids" is not particularly limited, but means about, for example, 1 to 20, preferably 1 to 10, more preferably 1 to 7, even more preferably 1 to 5, or especially preferably from 1 to 3.

The protein of the present invention can have an activity for catalyzing an α-1,4-glucosyl transfer reaction in addition to the activity for catalyzing an α-1,6-glucosyl transfer reaction. In this description, "activity for catalyzing an α-1,4-glucosyl transfer reaction" or "α-1,4-glucosyl transfer activity" refers to an activity for catalyzing a sugar transfer reaction that forms an α-1,4-glycosidic linkage. The a-1,4-glucosyl transfer activity can be evaluated by reacting a protein having an α-1,4-glucosyl transfer activity with any of maltose, maltotriose maltotetraose, and maltopentaose as a substrate, and detecting saccharides extended with glucoses transferred from the substrate with α-1,4-linkages in the resulting reaction product. The protein of the present invention can further have an activity for catalyzing an α-1,4-/α-1,6-glucoside linkage hydrolysis reaction, but expression of the α-1,4 -glucoside linkage hydrolysis reaction may be limited. As used in this description, "activity for catalyzing an α-1 ,4-/α-1 ,6-glucoside linkage hydrolysis reaction" or "α-1 ,4-/α-1,6-glucoside linkages hydrolysis activity" is an activity for catalyzing hydrolysis reactions that cleave α-1,4-/α-1,6-glucoside linkages.

The protein of the present invention can be produced as a recombinant protein in *Bacillus subtilis,* as described below. In general, the production of recombinant proteins using *Bacillus subtilis* as a host can enjoy the advantage of secretory expression, and the culture supernatant can be used for enzymatic reactions as it is without disrupting the bacterial cells. Therefore, it is used for the production of enzymes for use in an industrial scale. However, depending on the type of recombinant protein, expression of the enzyme activity in the culture supernatant differs. The protein of the present invention can produce an α-1,6-glucan more efficiently compared with the conventional enzymes having an α-1,6-glucosyl transfer activity, even when it is used as a culture supernatant of a transformed *Bacillus subtilis.*

The examples described later indicate that, when a culture supernatant of a transformed *Bacillus subtilis* is used in the reaction, the protein of the present invention can produce an α-1,6-glucan more efficiently compared with the enzyme having an a-1,6-glucosyl transfer activity derived from *Thermoanaerobacter siderophilus* described in Patent document 8.

The protein of the present invention may be a protein synthesized by chemical synthesis or a recombinant protein produced by a genetic recombination technique. The production of a recombinant protein will be explained below.

(a) A protein consisting of the amino acid of SEQ ID NO: 3, (b) a protein consisting of an amino acid sequence having an amino acid sequence identity of 90% or higher to the amino acid of SEQ ID NO: 3, and (c) a protein consisting of an amino acid sequence in which one or several amino acid(s) have been substituted, inserted, deleted and/or added in the amino acid sequence of SEQ ID NO: 3 can be prepared by genetic engineering techniques. For example, a gene encoding the amino acid sequence of SEQ ID NO: 3 can be produced by transforming a host cell with the gene as a DNA molecule that can replicate in a host cell, or a DNA molecule that can be incorporated into a chromosome and contains the gene in an expressible state, especially in a form thereof inserted into an expression vector, and culturing the host cell. Such a DNA molecule can be obtained by incorporating a polynucleotide encoding the amino acid sequence of SEQ ID NO: 3 or the like into a vector molecule. According to a preferred embodiment of the present invention, this vector is a plasmid. The preparation of DNA molecules in the present invention can be performed according to the methods described in Molecular Cloning: A Laboratory Manual 2nd Ed. (Sambrook, Maniatis, et al., Cold Spring Harbor Laboratory Press (1989)).

The vectors that can be used in the present invention can be selected from viruses, plasmids, cosmid vectors, and so forth, taking into consideration the type of host cell to be used. For example, examples include plasmids of the pJEXOPT2 series (see Japanese Patent Unexamined Publication (Kokai) No. 2009-17841), and plasmids of the pHT series for *Bacillus subtilis* as the host cell; bacteriophages of the λ phage type and plasmids of the pET series, pUC series, pCold series, and pGEX series for *E. coli* as the host cell; vectors of the YEp, YCp, and YIP series, and pLeu4, pPPLeu4, pJPLeu series (described in Japanese Patent Unexamined Publication (Kokai) No. No. Hei 4-218382) for yeast as the host cell, and so forth, but are not limited to these. The plasmid may contain a marker for selecting transformants, and the selection marker may be a drug resistance marker or a nutrient requirement marker gene, but is not limited to these.

Furthermore, the expression vector that can be used in the present invention can have DNA sequences necessary for expression of the protein gene, for example, promoter, terminator, ribosome binding site, transcriptional regulatory signal such as transcription termination signal, and translational regulatory signal. As the promoter, promoters of subtilisin, SPAC genes, and so forth can be used in *Bacillus subtilis*, and promoters of alcohol dehydrogenase gene (ADH), acid phosphatase gene (PHO), galactose gene (GAL), glyceraldehyde triphosphate dehydrogenase gene (GAP), and so forth can be used in yeast, but it is not limited to these. It is preferable to use a signal peptide, because it has an advantage that it makes the protein to be secreted out of the bacterial cells, and therefore increases productivity. The signal peptide can be replaced with one derived from *Bacillus subtilis* or yeast (e.g., invertase signal, acid phosphatase signal, λ-factor signal, etc.). In addition, in *E. coli,* more efficient expression can be devised by expressing molecular chaperone at the same time by using the cspA promoter or the like in addition to the commonly used lac promoter and T7 promoter.

For the culture of the transformed host cells, general methods for the used host cells can be used. Proteins are usually generated and accumulated in the cells or extracellular culture medium after about 1 to 4 days of culture. As for the culture conditions (medium, pH, temperature, etc.), the culture temperature, for example, is generally 25 to 37°C for bacteria, 25 to 30°C for yeast, and 37°C for eukaryotic cells. For the culture conditions, Manual of Gene Expression Experiments (Kodansha) and so forth can be referred to.

As the host cells, bacteria such as coli bacilli and hay bacilli, and yeast such as *Candida utilis, Saccharomyces cerevisiae* and *Pichia pastoris,* as well as *Rhizopus niveus, Rhizopus delemar,* and higher eukaryotes (such as CHO cells) can be used. As the hay bacilli, it is preferable to use a microorganism belonging to the genus *Bacillus.* It is known that *Bacillus* bacteria include strains that secrete proteins outside the cells of the bacteria (e.g., *Bacillus subtilis*). Strains that secrete almost no protease are also known, and it is also preferable to use such strains as the host. In the present invention, yeast, filamentous fungi or bacteria are preferred as the host cells, but bacteria are more preferred, and *Bacillus subtilis* is especially preferred.
As shown in the examples described later, when the gene of the sequence of SEQ ID NO: 4 was expressed by using *Bacillus subtilis* ISW1214 as a host, enzyme activity was observed in the culture supernatant and purified protein of *Bacillus subtilis* ISW1214.

The recombinant protein produced by the transformant can be isolated and purified by an appropriate combination of known separation and purification methods. These separation and purification methods include, for example, methods utilizing differences in solubility such as salt precipitation and solvent precipitation, methods utilizing differences in molecular weights such as dialysis, ultrafiltration, gel filtration and SDS-polyacrylamide electrophoresis, methods utilizing differences in electric charges such as ion exchange chromatography, methods utilizing differences in hydrophobicity such as hydrophobic chromatography and reverse phase chromatography, and methods utilizing differences in isoelectric point such as isoelectric focusing, as well as affinity chromatography, and so forth. In addition to the purification methods described in the examples, for example, Basic Experimental Methods for Proteins and Enzymes (Nankodo) can be referred to for general separation and purification methods.

The present invention provides a protein derived from *Tepidibacillus decaturensis* and having the following properties:
(1) the protein has an activity for catalyzing an a-1,6-glucosyl transfer reaction;
(2) the molecular weight measured by SDS-PAGE is from 95,000 to 105,000;
(3) the optimum pH is 3.9 to 4.6;
(4) the stable pH range is 4.2 to 9.0;
(5) the optimum temperature is 50 to 55°C; and
(6) the temperature stability is maintained at 50°C or lower.

The protein derived from *Tepidibacillus decaturensis* of the present invention has a molecular weight of about 99,000 as measured by SDS-PAGE. In this description, the molecular weight is mentioned for a protein expressed and secreted with a gene recombined in a plasmid by *Bacillus subtilis* as a host cell. The protein of which molecular weight is measured to be 99,500 in Example 1 is a protein having a His-tag (six histidines were added) at the C-terminal side, and not having the secretory signal consisting of estimated 17 amino acids, which had been cleaved. When the protein is secreted out of the bacterial cells, the secretory signal is cleaved, but the cleavage site may slightly shift. Although the actual cleavage site has not been confirmed, it is assumed that the cleavage site is in the range of several amino acids from the amino acid showing a high score for cleavage site predicted by the signal peptide prediction server SignalP4.1 (http://www.cbs.dtu.dk/services/SignalP/). In a preferred embodiment, the protein of the invention has a molecular weight in the range of 95,000 to 105,000 as measured by SDS-PAGE.

When measured at a temperature of 40°C, the protein of the present invention shows the maximum activity at pH 4.2, and the optimum pH is 3.9 to 4.6. In addition, the protein of the present invention was stable at pH 4.2 to 9.0 in a test in which the protein was maintained at a temperature of 4°C for 24 hours. The optimum pH and pH stability were determined by measuring maltose degradation activity under the conditions shown in Example 3, Sections 1 and 2.

When measured at pH 6.0, the protein of the present invention shows the maximum activity at a temperature of 55°C, and the optimum temperature is 50 to 55°C. In addition, the protein of the present invention was stable at a temperature of 50°C or lower in a test where the protein was maintained at pH 6.0 for 60 minutes. The optimum temperature and temperature stability were determined by measuring maltose degradation activity under the conditions shown in Example 3, Sections 3 and 4.

The optimum pH, optimum temperature, pH stability, and temperature stability of the protein of the present invention were determined by measuring maltose degradation activity based on the amount of glucose produced from maltose as a substrate. With the protein of the present invention, the degradation of maltose to glucose may be achieved by an α-1,4-glucosyl transfer reaction, a-1,6-glucosyl transfer reactions, and α-1,4-glucoside linkage hydrolysis reaction. Since it is considered that substantially the same catalytic site is involved in the 1,4-glucosyl transfer activity, a-1,6-glucosyl transfer activity, and α-1,4-glucoside linkage hydrolysis activity, it is considered that all the activities show similar reaction characteristics (optimum pH, optimum temperature, and pH and temperature stabilities), and therefore the maltose degradation activity was used for evaluating the reaction characteristics.

The protein of the present invention can act on, but not limited to, maltooligosaccharides, isomaltooligosaccharides, and starch partial degradation products as substrates. Maltooligosaccharides and isomaltooligosaccharides include, but are not limited to, maltose, maltotriose, maltotetraose, maltopentaose, maltohexaose, maltoheptaose, maltooctaose, isomaltose, isomaltotriose, isomaltotetraose, isomaltopentaose, isomaltohexaose, isomaltoheptaose, isomaltooctaose, panose, and isopanose. The starch partial degradation products will be described later.

### (Culture supernatant)

The present invention provides a culture supernatant of a transformed cell into which any of the polynucleotides (a) to (c) mentioned below has been introduced:
(a) a polynucleotide consisting of the nucleotide sequence of SEQ ID NO: 4;
(b) a polynucleotide that hybridizes with a complementary strand of a polynucleotide consisting of the nucleotide sequence of SEQ ID NO: 4 under stringent conditions; and
(c) a polynucleotide having a sequence identity of 90% or higher to the polynucleotide consisting of the nucleotide sequence of SEQ ID NO: 4.

The nucleotide sequence of SEQ ID NO: 4 is a nucleotide sequence encoding an amino acid sequence corresponding to the amino acid sequence SEQ ID NO: 1 obtained from the genome information of the microaerophilic bacterium *Tepidibacillus decaturensis* (NCBI Reference Sequence: WP_068722961.1), of which signal peptide is deleted (SEQ ID NO: 3).

A culture supernatant of a transformed cell that is introduced with a polynucleotide that hybridizes with a complementary strand of the polynucleotide consisting of the nucleotide sequence of SEQ ID NO: 4 under stringent conditions and encodes a protein that exhibits an α-1,6-glucosyl transfer activity may also be included in the scope of the present invention. The term "polynucleotide that hybridizes with a complementary strand of the polynucleotide consisting of the nucleotide sequence of SEQ ID NO: 4 under stringent conditions" means a polynucleotide (e.g., DNA) that can be obtained by using a complementary strand of the polynucleotide consisting of the nucleotide sequence of SEQ ID NO: 4 as a probe in the colony hybridization method, plaque hybridization method, Southern blot hybridization method, or the like. The "stringent conditions" means, for example, such conditions that DNA or the like can be identified by hybridization at 65°C in the presence of 0.7 to 1.0 M sodium chloride, and following washing of the filter at 65°C with a 0.1 to 5 x SSC solution (composition of 1 x SSC: 150 mM sodium chloride, 15 mM sodium citrate) (Molecular Cloning: A Laboratory Manual 2nd Ed. (Sambrook, Maniatis et al., Cold Spring Harbor Laboratory Press (1989)).

A culture supernatant of a transformed cell introduced with a polynucleotide having a sequence identity of 90% or higher, 91% or higher, 92% or higher, 93% or higher, 94% or higher, 95% or higher, 96% or higher, 97% or higher, 98% or higher, or 99% or higher to the polynucleotide consisting of the nucleotide sequence of SEQ ID NO: 4 and encoding a protein showing an a-1,6-glucosyl transfer activity may also be included in the scope of the present invention. The sequence identity is defined as the percentage of nucleotides residues that are identical between two aligned nucleotide sequences to be compared, wherein gaps are introduced in order to obtain the maximum sequence identity, if necessary. The nucleotide sequence identity can be determined by using publicly available computer software such as, for example, BLAST, BLAST-2, ALIGN, and Megalign (DNASTAR) software.

The transformed cell described above is preferably that of *Bacillus subtilis.* The culture supernatant of the present invention is substantially free from cells, and the cells can be removed with a filter or by centrifugation.

The culture supernatant of the present invention has an activity for catalyzing an a-1,6-glucosyl transfer reaction. The culture supernatant of the present invention can be used as it is for the reaction to produce an α-1,6-glucan, without disrupting the bacterial cells. A culture supernatant in which the gene derived from *Thermoanaerobacter siderophilus* described in Patent document 8 is expressed and secreted using *Bacillus subtilis* as a host cell can also be used for producing an α-1,6-glucan. In comparison with such a supernatant, the culture supernatant of the present invention in which the gene derived from *Tepidibacillus decaturensis* is expressed and secreted by using *Bacillus subtilis* as a host cell can more efficiently produce an α-1,6-glucan. Specifically, in the examples described later, it was demonstrated that the protein of the present invention can produce the same amount of an α-1,6-glucan with an amount corresponding to one-fourth of the addition amount of the enzyme derived from *Thermoanaerobacter siderophilus* mentioned in Patent document 8, which was used as a control. Furthermore, the α-1,6-glucosyl transfer reaction catalyzed by the protein of the present invention reached around the plateau of the reaction in 48 hours, and therefore it was suggested that it can produce an α-1,6-glucosyl transfer reaction product in a shorter time.

### (Enzyme preparation)

The present invention provides an enzyme preparation for use in production of an α-1,6-glucan from an oligosaccharide and/or polysaccharide having an α-1,4-glucosidic linkage and/or an α-1,6-glucosidic linkage, which contains the aforementioned protein having an activity for catalyzing an α-1,6-glucosyl transfer reaction and/or the aforementioned culture supernatant. By contacting the enzyme preparation of the present invention with an oligosaccharide and/or polysaccharide having an α-1,4-glucosidic linkage and/or an α-1,6-glucosidic linkage in a reaction mixture under conditions preferred for expression of the a-1,6-glucosyl transfer reaction of the protein, glucose is transferred and used for extension with α-1,6 linkage by the α-1,6-glucosyl transfer activity of the protein, and an α-1,6-glucan is obtained.

Examples of the oligosaccharide and/or polysaccharides having an α-1,4-glucosidic linkage and/or an α-1,6-glucosidic linkage as the substrate for the present invention include maltooligosaccharides and dextrins. In this description, "oligosaccharide" means a carbohydrate in which 2 to 10 monosaccharide molecules are linked through glucosidic linkages, and "polysaccharide" means a carbohydrate in which many monosaccharide molecules, specifically 10 or more monosaccharide molecules, are polymerized through glucosidic linkages. Maltooligosaccharides are carbohydrates with a degree of polymerization of from 2 to 10, in which glucoses are linked through α-1,4 linkages, and include maltose, maltotriose, maltotetraose, maltopentaose, maltohexaose, maltoheptaose, maltooctaose, and so forth. Isomaltooligosaccharides are carbohydrates with a degree of polymerization of from 2 to 10, consisting of glucoses linked through α-1,4 linkages and α-1,6 linkages, or glucoses linked only through α-1,6 linkages, and include isomaltose, panose, isomaltotriose, isomaltotetraose, isopanose, isomaltopentaose, isomaltohexaose, isomaltoheptaose, isomaltooctaose, and so forth. Dextrins are obtained by partial hydrolysis of starch, and are obtained as mixtures of carbohydrates of various degrees of polymerization, in which glucoses are linked through α-1,4 linkages and α-1,6 linkages. In general, those of a dextrose equivalent (DE) in the range not lower than 10 and not higher than 20 and those of DE lower than 10 may be distinguished as maltodextrins and dextrins, respectively; however, as used in this description, "dextrin" means a low molecular weight version of starch, regardless of the value of DE, and the term is used to mean a concept including maltodextrins.

When an α-1,6-glucan is produced by using the enzyme preparation of the present invention or the composition of the present invention described below, a starch partial degradation product can be used as a starting material. This is because the starch partial degradation product consists mainly of dextrins, and partially of maltooligosaccharides and isomaltooligosaccharides. The partial starch degradation product can be obtained by hydrolyzing starch with an acid or enzyme, and performing separation and purification as necessary.

The α-1,6-glucan produced by using the enzyme preparation of the present invention or the composition of the present invention described later may be an oligosaccharide or polysaccharide having a glucose polymerization degree of 2 or higher, comprising glucoses as a constituent sugar, and having an α-1,6- glucosidic linkage. That is, the α-1,6-glucan may also have α-1,2-, α-1,3-, and α-1,4-glucosidic linkages in addition to the α-1,6- glucosidic linkage. Details of the α-1,6-glucan of the present invention will be described below.

### (Composition)

The present invention provides a composition containing any of the proteins (a) to (c) mentioned below and having an activity for catalyzing an a-1,6-glucosyl transfer reaction:
(a) a protein consisting of the amino acid sequence of SEQ ID NO: 3;
(b) a protein consisting of an amino acid sequence having an amino acid sequence identity of 90% or higher to the amino acid sequence of SEQ ID NO: 3; and
(c) a protein consisting of an amino acid sequence in which one or several amino acid(s) have been substituted, inserted, deleted and/or added in the amino acid sequence of SEQ ID NO: 3.

The protein consisting of the amino acid sequence of SEQ ID NO: 3 contained in the above composition is the same as that described above (described for the enzyme preparation). For example, the protein contained in the above composition may be a protein consisting of an amino acid sequence having an amino acid sequence identity of 90% or higher, 91% or higher, 92% or higher, 93% or higher, 94% or higher, 95% or higher, 96% or higher, 97% or higher, 98% or higher, or 99% or higher to the amino acid sequence of SEQ ID NO: 3, and exhibiting an α-1,6-glucosyl transfer reaction activity. Further, the protein contained in the aforementioned composition may be a protein consisting of an amino acid sequence in which one or several amino acid(s) have been substituted, inserted, deleted and/or added in the amino acid sequence of SEQ ID NO: 3, and having an a-1,6-glucosyl transfer reaction activity.

The protein contained in the above composition may be derived from *Tepidibacillus decaturensis* and may have any of the following properties:
(1) the protein has an activity for catalyzing an a-1,6-glucosyl transfer reaction;
(2) the molecular weight measured by SDS-PAGE is from 95,000 to 105,000;
(3) the optimum pH is 3.9 to 4.6;
(4) the stable pH range is 4.2 to 9.0;
(5) the optimum temperature is 50 to 55°C; and
(6) the temperature stability is maintained at 50°C or lower.

The aforementioned composition can be used for production of an α-1,6-glucan from an oligosaccharide and/or polysaccharide having an α-1,4-glucosidic linkage and/or an α-1,6-glucosidic linkage. An α-1,6-glucan can be produced by the method for producing an α-1,6-glucan described below.

In addition to the aforementioned protein, the composition of the present invention may further contain a further component or components so long as such components do not inhibit the α-1,6-glucosyl transfer reaction. These may be, for example, components used in ordinary enzyme compositions, such as buffer, stabilizer, and excipient. Such further components are known from the prior art, and are well known to those skilled in the art. Form of the composition of the present invention is not also limited, and may be a solid (e.g., powdery form) or liquid. The composition of the present invention can be used, for example, by adding the composition in a solid or liquid form to a solution of a substrate.

### (Method for producing α-1,6-glucan)

The method for producing an α-1,6-glucan of the present invention comprises a reaction step of allowing the enzyme preparation or composition of the present invention to act on an oligosaccharide and/or polysaccharide having an α-1,4-glucosidic linkage and/or an α-1,6-glucosidic linkage to obtain an α-1,6-glucan. If the enzyme preparation or composition of the present invention is contacted with an oligosaccharide and/or polysaccharide having an α-1,4-glucosidic linkage and/or an α-1,6-glucosidic linkage in a reaction solution under conditions suitable for the expression of the a-1,6-glucosyl transfer activity, glucose is transferred so that the sugar chain is extended with α-1,6 linkages by the a-1,6-glucosyl transfer activity, and thereby an α-1,6-glucan can be produced.

Examples of the oligosaccharide and/or polysaccharides having an α-1,4-glucosidic linkage and/or an α-1,6-glucosidic linkage, which is the substrate used in the method for producing an α-1,6-glucan of the present invention, include maltooligosaccharides, isomaltooligosaccharides and dextrins.

Maltooligosaccharides, isomaltooligosaccharides and dextrins are as described above. The maltooligosaccharides and isomaltooligosaccharides may be of high purity reagent level, or of low purity like maltooligosaccharide syrup. Maltooligosaccharides that can be used for the present invention include, but are not limited to, Fujioligo #360, Fujioligo #450 (Nihon Shokuhin Kako Co., Ltd.), and so forth. Isomaltooligosaccharides usable for the present invention include, but not limited to, Isomalto 500, Isomalto 900 (Showa Sangyo Co., Ltd.), and so forth. Dextrins usable in the present invention include, but not limited to, Pinedex #1, Pinedex #2, Pinedex #4, Pinedex #6, Pinedex #100 (Matsutani Chemical Industry Co., Ltd.), and so forth. The substrate concentration can be, but not limited to, in the range of 0.1 to 40% (w/w) in the reaction solution. Since a higher substrate concentration provides a higher glycosyl transfer activity, a higher substrate concentration is preferred from the viewpoint of obtaining a higher concentration of an α-1,6-glucan. A higher substrate concentration is also preferred from the viewpoint that heat resistance of enzymes is generally improved in the presence of the substrate.

The method for producing an α-1,6-glucan of the present invention may, if necessary, comprise a step of hydrolyzing starch to obtain the oligosaccharide and/or polysaccharide having an α-1,4-glucosidic linkage and/or an α-1,6-glucosidic linkage, which is performed prior to the reaction step described above. In this step, starch is hydrolyzed with an acid or enzyme, separated and purified as required according to a conventional method to obtain a maltooligosaccharide, isomaltooligosaccharide, and dextrin, and these are the oligosaccharide and/or polysaccharide having an α-1,4-glucosidic linkage and/or an α-1,6-glucosidic linkage. In this step, degree of the hydrolysis of the starch can be appropriately adjusted. The degree of degradation can be expressed in terms of dextrose equivalent (DE). The DE of the of starch hydrolysate obtained in this step may be, but not limited to, from 2 to 70, which is DE suitable for the degree of polymerization of the α-1,6-glucan desired to be produced in the subsequent reaction step. In addition, a short chain length amylose can also be used as the starch hydrolysate. Further, as the starch hydrolysate, a liquefying liquid obtained by treating common starches (e.g., corn starch, potato starch, sweet potato starch, wheat starch, rice starch, tapioca starch, etc.) with an acid or enzyme (e.g., α-amylase, etc.) can also be used.

By the method for producing an α-1,6-glucan of the present invention, an α-1,6-glucan having a degree of polymerization of 2 to 30 can be produced. Such an α-1,6-glucan having a degree of polymerization of 2 to 30 is produced as a mixture of saccharides dominantly containing α-1,6-glucans having a degree of polymerization of 2 to 30. The composition of this mixture varies depending on the various reaction conditions, such as type and concentration of the substrate, reaction time, whether or not another enzyme is used, and if used, type of the other enzyme used. In a preferred embodiment, the composition of the α-1,6-glucan-containing composition produced according to the present invention can contain 70% or more, especially 80% or more, of saccharides having a DP of 3 to 30. In a preferred embodiment, the composition of the α-1,6-glucan-containing composition produced according to the present invention can contain 30% or more, especially 40% or more, of saccharides having a DP of 10 to 20. Further, the composition of the α-1,6-glucan-containing composition produced according to the present invention can contain 30% or more, especially 40% or more, further preferably 50% or more, of saccharides having a DP of 10 to 30.

The α-1,6-glucan that can be produced according to the present invention may be an isomaltooligosaccharide and/or isomaltomegalosaccharide. Isomaltooligosaccharides are carbohydrates having a degree of polymerization of from 2 to 10 in which glucoses are linked with linkage schemes including the α-1,6 linkage, and isomaltomegalosaccharides are carbohydrates having a degree of polymerization of from 10 to 100 in which glucoses are linked with linkage schemes including the α-1,6 linkage. The α-1,6-glucan that can be produced according to the present invention is preferably an isomaltoligosaccharide and/or isomaltomegalosaccharide having a degree of polymerization of 2 to 30. By using the enzyme preparation or composition of the present invention, and adjusting the type and concentration of the substrate, the reaction conditions, and so forth, an isomaltooligosaccharide and/or isomaltomegalosaccharide having a desired degree of polymerization can be produced.

In a preferred embodiment, by the method for producing an α-1,6-glucan of the present invention, an α-1,6-glucan having a high content of carbohydrates having a degree of polymerization of 3 to 20 can be produced. In this case, the substrate is preferably a maltooligosaccharide or isomaltooligosaccharide having a degree of polymerization of 3 to 10, and a maltooligosaccharide or isomaltooligosaccharide having a degree of polymerization of 5 to 10 is particularly preferred. In another preferred embodiment, by the production method of the present invention, an α-1,6-glucan having a high content of carbohydrates having a degree of polymerization of 3 to 20 can be produced using a dextrin as a substrate. In this case, the substrate is preferably a dextrin having a DE of 3 to 20, and a dextrin having a DE of 4 to 10 is more preferred. In yet another preferred embodiment, by the production method of the present invention, an α-1,6-glucan having a high content of carbohydrates having a degree of polymerization of 3 to 20 can be produced.

In the reaction step of the method for producing an α-1,6-glucan of the present invention, the enzyme preparation or composition of the present invention and another enzyme can be used in combination. The other enzyme can be used together with the enzyme preparation or composition of the present invention in order to, but are not limited to, increase the starch partial degradation products that can be substrates for the enzyme preparation or composition, and increase the efficiency of glucose transfer and chain extension with α-1,6 linkages and the yield of the α-1,6-glucan.

The other enzyme can be used in order to, but not limited to, cleave α-1,4 linkages and α-1,6 linkages of an oligosaccharide and/or polysaccharide having an α-1,4-glucosidic linkage and/or α-1,6-glucosidic linkage. In particular, when the degree of degradation of the starch partial degradation product is low, it can be used to cleave α-1,4 linkages and α-1,6 linkages of the saccharide having a high degree of polymerization. This is because, although it is not intended to be bound by any specific theory concerning the present invention, it is considered that cleavage of the α-1,4 linkages and α-1,6 linkages of the starch partial degradation product increases starch partial degradation products that can serve as the substrate for the enzyme preparation or composition, and increases efficiency of the glucose transfer and chain extension with α-1,6 linkages caused by the enzyme preparation or composition.

Examples of the other enzyme include, but are not limited to, α-amylase (e.g., Kleistase (registered trademark) L-1), isoamylase, and/or pullulanase. The origin, preparation method, and so forth of the α-amylase, isoamylase, and pullulanase used in the present invention are not particularly limited. Alpha-amylase is an enzyme that cleaves α-1,4 linkages of starch or the like to degrade it into polysaccharides, oligosaccharides, and maltose. Isoamylase is an enzyme that cleaves α-1,6 linkages of starch or the like, and pullulanase is an enzyme that cleaves α-1,6 linkages of pullulan (polysaccharide containing repeats of α-1,4 linkage, α-1,4 linkage, and α-1,6 linkage). By allowing the enzyme preparation or composition of the present invention together with the other enzyme mentioned above to act on the starch partial degradation product, an α-1,6-glucan can be efficiently obtained with a high yield.

The concentrations (number of units) of the enzyme preparation or compositions of the invention, and α-amylase, isoamylase, and/or pullulanase when used in combination in the reaction solution used in the reaction step described above can be appropriately determined in consideration of type of the starch partial degradation product that is the substrate (i.e., degree of degradation), concentration thereof in the reaction solution, length of the reaction time, and so forth. Furthermore, when another enzyme is used in combination, the concentration ratios of the enzyme preparation or composition of the present invention, α-amylase, isoamylase, and pullulanase can also be appropriately determined in accordance with the type of the starch partial degradation product that is the substrate, and origins and performances of the enzymes.

The reaction temperature of the reaction step described above is not particularly limited so long as it is in a temperature range in which the enzyme preparation or composition of the present invention stably acts. In order to increase the efficiency of α-1,6-glucan synthesis, it is desirable to set the reaction temperature to be in the temperature range where the protein having the activity for catalyzing the α-1,6-glucosyl transfer reaction of the present invention acts more efficiently.
The protein of the present invention is stable at a temperature of 50°C or lower in the absence of a substrate. Accordingly, the temperature of the reaction system in which the enzyme preparation or composition of the present invention is used (in the presence of a substrate) can be set to be in the temperature range of, for example, 35 to 60°C. Although precise temperature control may be difficult in an industrial scale reaction system, the enzyme preparation or composition is advantageous in that it can be stably used in a wide temperature range.

The above reaction step can be carried out at a reaction temperature of 35 to 60°C, but 45 to 55°C is preferred. This is because the optimum temperature of the protein of the present invention is 55°C, at which it shows high activity.

When another enzyme is used in combination, the reaction temperature of the reaction step described above may be in a temperature range in which the enzyme preparation or composition of the present invention, as well as α-amylase, isoamylase and/or pullulanase can stably act. Since the enzyme preparation or composition of the present invention is stable over a wide temperature range as described above, it is possible in many cases to set the temperature within such a range that activities of all enzymes used in the reaction system can be fully utilized, taking into account the temperature at which the other enzyme or enzymes used in combination are stably activated.

The reaction temperature may not be constant throughout the whole reaction period, and can be appropriately adjusted. For example, if it is desirable to increase the activity of another enzyme used in combination in an early stage of the reaction period, the temperature in the early stage of the reaction can be set to be in a temperature range in which the activity of the enzyme is increased, and in the middle or late stage of the reaction period, the temperature can be set to be in a temperature range in which the activity of the enzyme preparation or composition of the present invention is increased.

The reaction pH of the reaction step described above is not particularly limited so long as it is in a pH range in which the enzyme preparation or composition of the present invention acts stably, and may be appropriately set in accordance with other conditions such as reaction temperature. In order to increase synthesis efficiency of the α-1,6-glucan, it is preferable to set the reaction pH in a pH range in which the protein having an activity for catalyzing the α-1,6-glucosyl transfer reaction of the present invention acts more efficiently. Since the protein having an activity for catalyzing an α-1,6-glucosyl transfer reaction of the present invention shows the maximum activity at pH 4.2, and the optimum pH thereof is 3.9 to 4.6, the reaction pH of the above reaction step can be set to be 3.9 to 4.6, but is not limited to this range.

The reaction time of the above reaction step can be appropriately determined in consideration of the reaction temperature, concentration of the substrate, and when another enzyme is used in combination, activity of the enzyme used. On the basis of the conventional techniques in this field, a suitable reaction time for producing an α-1,6-glucan can be appropriately determined.

By the reaction of the above reaction step, an aqueous solution containing an α-1,6-glucan can be obtained. From this aqueous solution, the α-1,6-glucan can be purified by precipitation with an organic solvent using ethanol or the like, chromatographic fractionation, or treatment with an ultrafiltration membrane. By using each of these methods or any combination thereof, the α-1,6-glucan can be more efficiently purified.

The method for producing a food, feed, feed for fish culture, cosmetic, or medicament of the present invention comprises the step of: allowing the enzyme preparation or composition of the present invention to act on an oligosaccharide and/or polysaccharide having an α-1,4-glucosidic linkage and/or an α-1,6-glucosidic linkage to produce and thereby obtain an α-1,6-glucan, and the step of obtaining a food product, feed, feed for fish culture, cosmetic, or medicament by using the α-1,6 - glucan obtained in the foregoing step.

The step of allowing the enzyme preparation or composition of the present invention to act on an oligosaccharide and/or polysaccharide having an α-1,4-glucosidic linkage and/or an α-1,6-glucosidic linkage to produce and thereby obtain an α-1,6-glucan can be carried out according to the method for producing an α-1,6-glucan described above.

In the step of obtaining a food, feed, feed for fish culture, cosmetic, or medicament using the α-1,6-glucan obtained in the above step, the food, feed, feed for fish culture, cosmetic, or medicament can be produced by using the α-1,6-glucan of the present invention as one of the starting materials, or the α-1,6-glucan itself can be prepared in an appropriate form (powder, liquid, etc.), and provided as a food, feed, feed for fish culture, cosmetic, or medicament.

The method for producing a glycoside of the present invention is a method for producing a glycoside comprising the step of allowing the enzyme preparation or composition of the present invention to act on a sugar receptor and a sugar donor. A microorganism expressing the protein of the present invention may also be allowed to act on a sugar acceptor and sugar donor. By allowing the enzyme preparation or the composition to act on a solution containing a sugar donor and a sugar acceptor, a glycoside consisting of the sugar acceptor and at least one glycosyl group transferred to the sugar acceptor can be produced. The method for producing a glycoside of the present invention can be carried out by the method for producing an α-1,6-glucan described above with appropriately setting the reaction temperature and pH. In the method for producing a glycoside of the present invention, the reaction temperature can be adjusted according to the properties of the sugar acceptor. Since the enzyme preparation or composition of the present invention can be stably used over a relatively wide temperature range, it can be used for the production of glycosides of a wide range of compounds.

The sugar donor may be any compound that can be glycosyl-transferred by the enzyme preparation or composition of the present invention. More specifically, examples include maltooligosaccharides, and maltose and maltotriose are preferred.

The glycoside produced by the method of the present invention can have glycosidic linkage at the binding site of the sugar moiety and non-sugar compound.

The sugar receptor may be any compound having a hydroxyl group to which a glycosyl group can be transferred by the enzyme preparation or composition of the present invention. Specifically, alcohols (e.g., ethanol, 1-propanol, 2-propanol, L-menthol, 1-butanol, and 2-butanol), polyols (e.g., glycerol, and propylene glycol), vitamins (e.g., L-ascorbic acid, retinol, inositol, and tocopherol), flavonoids (e.g., quercetin, catechin, rutin, and hesperidin), phenol derivatives (e.g., hydroquinone), and so forth can be used, and it is not particularly limited so long as it is a compound having a hydroxyl group. If the sugar acceptor is easily oxidized, it is also effective to add a reducing agent to the reaction system as necessary.

The glycoside produced with the enzyme preparation or composition of the present invention can be used as one of the starting materials for food, feed, feed for fish culture, cosmetic, or medicament, and the glycoside itself can also be provided as a food, feed, feed for fish culture, cosmetics, or medicament. The glycoside produced with the enzyme preparations or compositions of the present invention is easily dissolved in water, can exist as a solid powder at room temperature, and is stable in quality, and therefore it can be widely used in foods, drugs, cosmetics, and so forth.

Examples of the food produced by the method for producing a food of the present invention include, but are not limited to, various carbohydrate foods (bread, noodles, rice, rice cakes), various Japanese confectioneries (senbei, arare, okoshi, gyuhi, mochi, manju, dorayaki, uiro, bean paste, yokan, mizu-yokan, nishikidama, Castella sponge cake, amedama candy, etc.), various Western confectioneries (bread, biscuit, cracker, cookie, pie, doughnut, steamed cake, pudding, jelly, mousse, bavarois, custard cream, cream puff, waffle, sponge cake, chocolate, chewing gum, caramel, nougat, candy, syrups, etc.), various ice confectioneries (ice cream, sherbet, gelato, shaved ice, etc.), various paste-like foods (flower paste, peanut paste, margarine, fruit paste, etc.), various beverages (beverages containing fruit juice, fruit juice, vegetable juice, cider, ginger ale, isotonic beverages, amino acid beverages, jelly beverages, coffee beverages, green tea, black tea, oolong tea, barley tea, dairy beverages, lactobacillus beverages, cocoa, beer, low-malt beer, malt-free beer-like alcoholic beverage, non-alcoholic beverages, beer-flavored beverages, liqueur, chuhai, sake, fruit wine, distilled spirits, nutritional drinks, health drinks, powdered beverages, etc.), processed fruit and vegetable foods (jam, marmalade, syrup, Chinese sugar confectionaries, pickles, etc.), various dairy products (cheese, yogurt, butter, condensed milk, powdered milk, etc.), powdered food (powdered soup, powdered mousse, powdered jelly, powdered sweetener, etc.), nutritional foods, diet foods, nutritional foods for sports, liquid foods, semi-solid liquid foods, nursing care foods, swallowing foods, and so forth.

Examples of the feed and feed for fish culture produced by the method for producing a feed or feed for fish culture of the present invention include, but are not limited to, feeds and feeds for fish culture for livestock, poultry, fish and shellfish, and insects (honeybees, silkworms, etc.). Examples of the form thereof include, but are not limited to, powder, pellet, tablet, kneaded feed, capsule, and so forth.

Examples of the cosmetic produced by the method for producing a cosmetic of the present invention include, but are not limited to, moisturizer, beautifying agent, and so forth. Examples of the form thereof include, but are not limited to, milky liquid, cream, emulsion, and so forth.

Examples of the medicament produced by the method for producing a medicament of the present invention include, but are not limited to, anti-obesity agent, blood glucose level increase inhibitor, and so forth, and examples of the form thereof include, but are not limited to, tablet, powder, liquid, capsule, and so forth.

### Examples

The present invention will be more specifically explained with reference to the following examples. However, the present invention is not limited to these examples. In this description, unless especially stated, "%," "part," and so forth are mass-based, and numerical ranges are mentioned so as to include their end points. Further, unless especially stated, the operating procedures were performed according to the methods described in Molecular Cloning: A Laboratory Manual 2nd Ed. (Sambrook, Maniatis et al., Cold Spring Harbor Laboratory Press (1989)).

### Example 1: Extracellular expression using Bacillus subtilis as host (1)

### 1. Construction of expression plasmid

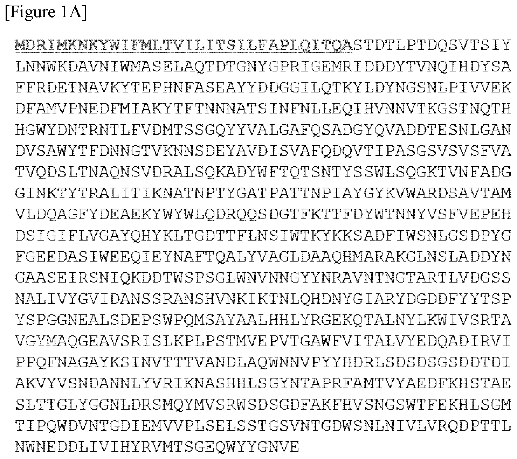

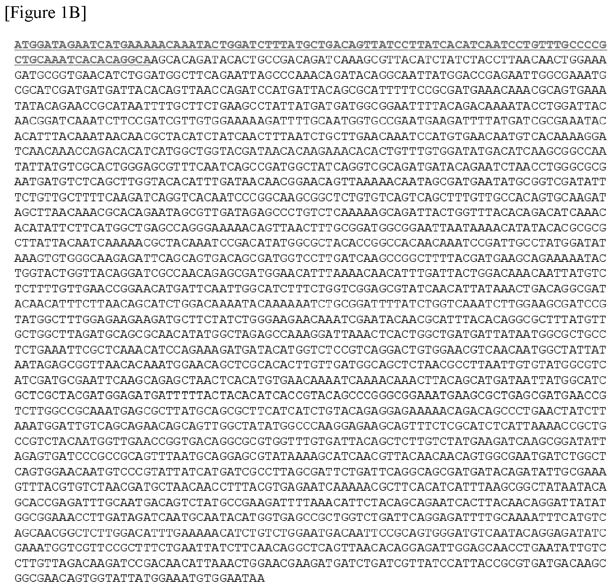

Various microorganisms were searched for novel enzymes, and the amino acid sequence derived from *Tepidibacillus decaturensis* (NCBI Reference Sequence: WP_068722961.1) was selected, and functional analysis thereof was conducted. The amino acid sequence thereof (SEQ ID NO: 1) and the nucleotide sequence (SEQ ID NO: 2) of the gene encoding the amino acid sequence (hereafter referred to as the objective gene) are shown in Fig. 1A and Fig. 1B, respectively. The amino acid sequence of SEQ ID NO: 3 corresponds to the amino acid sequence of SEQ ID NO: 1 of which signal peptide is deleted. The nucleotide sequence of SEQ ID NO: 4 corresponds to the nucleotide sequence of SEQ ID NO: 2 of which portion corresponding to the signal peptide is deleted. The nucleotide sequence of SEQ ID NO: 2 had been codon-corrected to optimize it for expression in *Bacillus subtilis.*

An expression plasmid for expressing the objective gene in *Bacillus subtilis* was constructed. First, the objective gene was amplified by PCR using a plasmid consisting of the pUC57 vector inserted with the nucleotide sequence of SEQ ID NO: 2 as a template, a primer containing a nucleotide sequence homologous to the end of the signal peptide sequence of the vector for sense strand amplification, and a primer containing a part of the His-Tag for anti-sense strand amplification. The PCR conditions are shown below. The total volume of the reaction solution for PCR amplification was 100 µL.

| | |
|---|---|
| 2 x Primestar Max Premix (Takara Bio) | 50 µL |
| 10 µM Primer (TdGH15A-Fw) | 2 µL |
| 10 µM Primer (TdGH15A-His-Rv) | 2 µL |
| 1 ng/µL Template | 2 µL |
| H₂O | 44 µL |

The primers used are shown in Table 1.

**[Table 1]**

| Primer | Sequence (5'→3') |
|---|---|
| TdGH15A-Fw (sense) | ACTGCTCTTGGATCCAGCACAGATACACTG (SEQ ID NO:5) |
| TdGH15A-His-Rv (anti-sense) | ATGGTGATGGTGGTGTTCCACATTTCCATAATACCACTG (SEQ ID NO:6) |

The PCR amplification reaction program was as follows. First, the reaction system was held at 96°C for 1 minute, followed by 35 cycles of 98°C for 10 seconds, 55°C for 5 seconds, and 72°C for 35 seconds, and further held at 72°C for 5 minutes. The obtained PCR product was subjected to agarose gel electrophoresis, the band corresponding to the amplified fragment (2,694 bp) was cut out from the gel, and the fragment was extracted and purified by using illustra^{™} GFX^{™} PCR DNA and Gel Band Purification Kit (GE).

To prepare a linearized plasmid for use in the In-Fusion (registered trademark) cloning reaction, PCR was performed by using a plasmid obtained by modification (addition of His-tag sequence to the C-terminus, etc.) of the vector pJEXOPT2 (see Japanese Patent Unexamined Publication (Kokai) Nos. 2009-17841 and 2009-17842) for optimization for this example as the template, and the primers shown in Table 2. For sense strand amplification, a primer including the His-tag sequence was used, and for anti-sense strand amplification, a primer designed for amplification from the end of the signal peptide sequence of the vector was used.

**[Table 2**

| Primer | Sequence (5'→3') |
|---|---|
| pJEXOPT2-His-Fw (sense) | CACCACCATCACCATCATTGAGTCGACCTGCAGATCTCTAGA (SEQ ID NO:7) |
| pJEXOPT2-Rv1 (anti-sense) | GGATCCAAGAGCAGTGGC (SEQ ID NO:8) |

The composition of the PCR amplification reaction solution was the same as that used for the amplification of the objective gene, except for the primers and template. The PCR amplification reaction program was as follows. First, the reaction system was held at 96°C for 1 minute, followed by 35 cycles of 98°C for 10 seconds, 55°C for 5 seconds, and 72°C for 35 seconds, and further held at 72°C for 5 minutes. The obtained PCR product was subjected to agarose gel electrophoresis, the band corresponding to the amplified fragment (6,953 bp) was cut out from the gel, and the fragment was extracted and purified by using Wizard SV Gel and PCR Clean-Up System. The amplified DNA fragment of the objective gene and the amplified fragment of the vector pJEXOPT2 were ligated by using In-Fusion HD Cloning Kit (Takara Bio). The ligation reaction was carried out by holding them at 50°C for 15 minutes.

*E. coli* DH5α was transformed with 2.5 µL of the ligation reaction solution, and a plasmid DNA was prepared from the culture medium in which the *E. coli* was cultured by using illustra^{™} plasmidPrep Mini Spin Kit (GE). The obtained plasmid was designated as "plasmid for expression of Protein 1 (His-tag added) in *Bacillus subtilis*".

### 2. Expression of recombinant protein

The plasmid for expression of Protein 1 (His-tag added) in *Bacillus subtilis* described above was introduced into *Bacillus subtilis* ISW1214 made into protoplasts (Takara Bio), and culture was performed at 30°C for 2 days in a regeneration agar medium (composition: 8.1% sodium succinate, 1% agar, 0.5% casamino acid, 0.5% yeast extract, 0.15% potassium dihydrogen phosphate, 0.35% dipotassium hydrogen phosphate, 0.5% glucose, 0.4% magnesium chloride, 0.01% bovine serum albumin, 0.001% methionine, and 0.001% leucine) containing 7.5 µg/mL tetracycline. The obtained colonies were cultured in a pre-culture medium and then in a main culture medium (cultured as described in Japanese Patent Unexamined Publication (Kokai) Nos. 2009-17841 and 2009-17842, provided that the compositions of the media were modified). The culture medium was centrifuged (15,000 x g, 4°C, 5 minutes), the supernatant was filtered through a 0.45 µm filter (Merck), and the filtrate was used as the culture supernatant.

### 3. Purification of recombinant protein

Affinity chromatography was carried out by the free fall method (open column) using a Ni column comprising Econo Column (BIO-RAD) packed with approximately 20 mL of Ni carrier (Chelating Sepharose Fast Flow (GE) on which Ni was bonded). After the column was equilibrated by passing a binding buffer (20 mM sodium dihydrogen phosphate, 500 mM sodium chloride, 30 mM imidazole, pH 7.4) in a volume corresponding to 4 times of the bed volume, 30 mL of the culture supernatant prepared in Example 1, "2. Expression of recombinant protein" was applied, and the binding buffer was further passed through the column (flow-through fraction). After the binding buffer was passed through in a volume corresponding to 5 times of the bed volume (washing fraction), the protein encoded by the objective gene was eluted by passing an elution buffer (20 mM sodium dihydrogen phosphate, 500 mM sodium chloride, 500 mM imidazole, pH 7.4) through the column in a volume corresponding to 5 times of the bed volume (elution fraction). Each fraction was subjected to SDS-PAGE to confirm the purity attained by the purification.

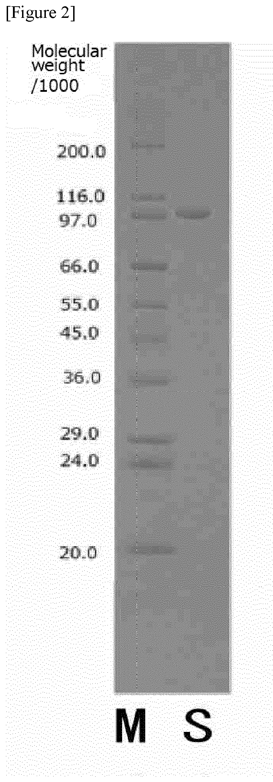

The elution fraction was concentrated by using Amicon Ultra 50K. The concentrated purified protein was dialyzed, the solution composition of the purified protein was substituted with 20 mM HEPES-NaOH (pH 7.0), and the solution was stored at 4°C. Absorbance at a wavelength of 280 nm and the amino acid sequence were entered into Nucleic and/or Amino Acid contents (http://www.gen-info.osaka-u.ac.jp/~uhmin/study/gc_content/index_en.html) to calculate the protein concentration. The purified protein was subjected to SDS-PAGE, and formation of a single band was confirmed (Fig. 2). The molecular weight was determined to be 99,500. The purified protein obtained by Example 1 is referred to as Protein 1 of the invention.

### Example 2: Extracellular expression using Bacillus subtilis as host (2)

### 1. Construction of expression plasmid

An expression plasmid for expressing the objective gene in *Bacillus subtilis* was constructed. First, the objective gene was amplified by PCR using a plasmid consisting of the pUC57 vector inserted with the nucleotide sequence of SEQ ID NO: 2 as a template, a primer containing a nucleotide sequence homologous to the end of the signal peptide sequence of the vector for sense strand amplification, and a primer containing a part of the terminator sequence for anti-sense strand amplification. The PCR conditions are shown below. The total volume of the reaction solution for PCR amplification was 100 µL.

| | |
|---|---|
| 2 x Primestar Max Premix (Takara Bio) | 50 µL |
| 10 µM Primer (TdGH15A-Fw) | 2 µL |
| 10 µM Primer (TdGH15A-Rv) | 2 µL |
| 1 ng/µL Template | 2 µL |
| H₂O | 44 µL |

The primers used are shown in Table 3.

**[Table 3]**

| Primer | Sequence (5'→3') |
|---|---|
| TdGH15A-Fw (sense) | ACTGCTCTTGGATCCAGCACAGATACACTG (SEQ ID NO:5) |
| TdGH15A-Rv (anti-sense) | GATCTGCAGGTCGACTTATTCCACATTTCCATA (SEQ ID NO:9) |

The PCR amplification reaction program was as follows. First, the reaction system was held at 96°C for 1 minute, followed by 35 cycles of 98°C for 10 seconds, 55°C for 5 seconds, and 72°C for 35 seconds, and further held at 72°C for 5 minutes. The obtained PCR product was subjected to agarose gel electrophoresis, the band corresponding to the amplified fragment (2,697 bp) was cut out from the gel, and the fragment was extracted and purified by using illustra^{™} GFX^{™} PCR DNA and Gel Band Purification Kit (GE).

To prepare a linearized plasmid for use in the In-Fusion (registered trademark) cloning reaction, PCR was performed by using a plasmid obtained by modification of the vector pJEXOPT2 (see Japanese Patent Unexamined Publication (Kokai) Nos. 2009-17841 and 2009-17842) for optimization for this example as the template, and the primers shown in Table 4. For anti-sense strand amplification, a primer designed for amplification from the end of the signal peptide sequence of the vector was used.

The primers used are shown in Table 4.

**[Table 4]**

| Primer | Sequence (5'→3') |
|---|---|
| pJEXOPT2-Fw (sense) | GTCGACCTGCAGATCTCTAG (SEQ ID NO:10) |
| pJEXOPT2-Rv2 (anti-sense) | GGATCCAAGAGCAGTGG (SEQ ID NO:11 ) |

The composition of the PCR amplification reaction solution was the same as that used for the amplification of the objective gene, except for the primers and template. The PCR amplification reaction program was as follows. First, the reaction system was held at 96°C for 1 minute, followed by 35 cycles of 98°C for 10 seconds, 55°C for 15 seconds, and 72°C for 35 seconds. The amplified product was subjected to agarose gel electrophoresis, and the band corresponding to the DNA fragment of the objective gene (6,953 bp) was cut out from the gel, the fragment was extracted and purified by using Wizard SV Gel and PCR Clean-Up System. The amplified DNA fragment of the objective gene and the amplified fragment of the vector pJEXOPT2 were ligated by using In-Fusion HD Cloning Kit (Takara Bio). The ligation reaction was carried out by holding them at 50°C for 15 minutes.

*E. coli* DH5α was transformed with 2.5 µL of the ligation reaction solution, and a plasmid DNA was prepared from the culture medium in which the *E. coli* was cultured by using illustra^{™} plasmidPrep Mini Spin Kit (GE). The obtained plasmid was designated as "plasmid for expression of Protein 2 in *Bacillus subtilis*".

### 2. Expression of recombinant protein

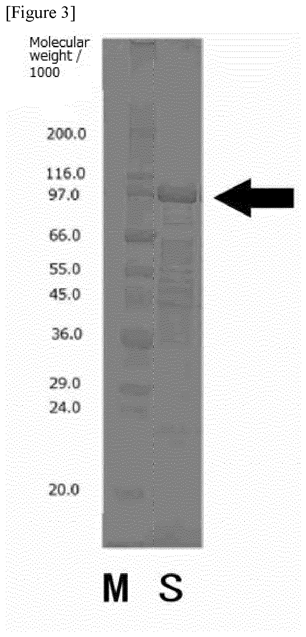

The plasmid for expression of Protein 2 in *Bacillus subtilis* described above was introduced into *Bacillus subtilis* ISW1214 made into protoplasts (Takara Bio), and culture was performed at 30°C for 2 days in a regeneration agar medium (composition: 8.1% sodium succinate, 1% agar, 0.5% casamino acid, 0.5% yeast extract, 0.15% potassium dihydrogen phosphate, 0.35% dipotassium hydrogen phosphate, 0.5% glucose, 0.4% magnesium chloride, 0.01% bovine serum albumin, 0.001% methionine, and 0.001% leucine) containing 7.5 µg/mL tetracycline. The obtained colonies were cultured in a pre-culture medium and then in a main culture medium for 70 hours (cultured as described in Japanese Patent Unexamined Publication (Kokai) Nos. 2009-17841 and 2009-17842, provided that the compositions of the media were modified). The culture medium was centrifuged (5,000 x g, 4°C, 5 minutes), the supernatant was filtered through a 0.45 µm filter (Merck), and the filtrate was used as the culture supernatant. This culture supernatant was subjected to SDS-PAGE, and it was confirmed that a band appeared around the target size (99,300) (Fig. 3). The protein obtained by Example 2 is referred to as Protein 2 of the invention.

### Example 3: Physicochemical properties of protein

In order to confirm the optimum pH, pH stability, optimum temperature, and temperature stability of Protein 1 of the invention obtained in Example 1, the maltose degradation activity of Protein 1 of the invention was measured as follows on the basis of the glucose production amount. One U of the enzyme activity unit is defined as the amount of the enzyme that produces 1 µmol of glucose in 1 minute of each reaction.

### 1. Optimum pH

To determine the optimum pH, the enzyme activity was measured by using 100 mM Britton-Robinson buffers of pH 2.5 to 11.0 (0.5 increments). To a mixture of 20 µL of 5 (w/v) % D-(+)-maltose, 20 µL of 100 mM Britton-Robinson buffer (pH 2.5 to 11.0), and 60 µL of ultrapure water, 10 µL of Protein 1 of the invention diluted to 40.3 µg/mL using Dilution buffer A (20 mM sodium acetate buffer (pH 6.0), BSA 1 mg/mL) was added, and the resulting mixture was maintained at 40°C for 30 minutes. Then, 100 µL of 2 M Tris-HCl (pH 7.0) was added to terminate the reaction. To 100 µL of the reaction-terminated solution, 100 µL of Glucose C-II Test Wako was added, and the resulting mixture was maintained at 37°C for 20 minutes. Then, the absorbance of the mixture was measured at 492 nm, and the amount of glucose produced by the reaction was determined by using a calibration curve prepared with glucose (0 to 0.008%). When the enzymatic activity of the protein was determined, the protein showed the maximum activity at pH 4.2, and showed the activity corresponding to 95% or more of the maximum activity at pH 3.9 to 4.6 (Fig. 4, black circles). The optimal pH was measured in triplicate (n = 3).

### 2. pH Stability

When the pH stability was evaluated, a mixture of 5 µL of 806 µg/mL Protein 1 of the invention, 10 µL of 100 mM Britton-Robinson buffer (pH 2.5 to 11.0), and 35 µL of ultrapure water was maintained at 4°C for 24 hours, and then the mixture was diluted with Dilution buffer B (200 mM sodium acetate buffer (pH 6.0), 1 mg/mL BSA) to a concentration of 16.1 µg/mL of Protein 1 of the invention, and used as a sample for each pH. The 806 µg/mL Protein 1 of the invention solution was diluted to a concentration of 16.1 µg/mL using Dilution buffer B, and used as the control.

To a mixture of 20 µL of 5 (w/v) % D-(+)-maltose, 20 µL of 100 mM sodium acetate buffer (pH 6.0), and 60 µL of ultrapure water, 10 µL of the sample for each pH or control was added, and the resulting mixture was maintained at 40°C for 30 minutes. Then, 100 µL of 2 M Tris-HCl (pH 7.0) was added to terminate the reaction. To 100 µL of the reaction-terminated solution, 100 µL of Glucose C-II Test Wako was added, and the resulting mixture was maintained at 37°C for 20 minutes. Then, the absorbance of the mixture was measured at 492 nm, and the amount of glucose produced by the reaction was determined to measure the residual activity. The residual activity was calculated as the ratio (%) of the activity of the sample for each pH to the activity of the control. When the pH stable range was defined as a range in which 70% or more of residual activity was observed, the pH stable range of the protein was 4.2 to 9.5 (Fig. 4, white circles). The pH range in which the protein showed the residual activity of 80% or more was 5.5 to 9.5, that for the residual activity of 90% or more was 6.5 to 9.0, and that for the residual activity of 95% or more was 7.5 to 8.5. The pH stability was measured in triplicate (n = 3).

### 3. Optimum temperature

When the optimum temperature is determined, the enzyme activity was measured at 30 to 80°C (5°C increments). To a mixture of 20 µL of 5% (w/v) D-(+)-maltose, 20 µL of 100 mM sodium acetate buffer (pH 6.0), and 60 µL of ultrapure water, 10 µL of Protein 1 of the invention diluted to 40.3 µg/mL using Dilution buffer A was added, and the resulting mixture was maintained at each temperature (30 to 80°C) for 30 minutes. Then, 100 µL of 2 M Tris-HCl (pH 7.0) was added to terminate the reaction. To 100 µL of the reaction-terminated solution, 100 µL of Glucose C-II Test Wako was added, and the resulting mixture was maintained at 37°C for 20 minutes. Then, the absorbance was measured at 492 nm, and the amount of glucose produced by the reaction was determined to measure the enzyme activity. As a result, the maximum activity was observed at 55°C, and the activity corresponding to 95% or more of the maximum activity was observed in the range of 50 to 55°C (Fig. 5, black circles). The optimal temperature was measured in triplicate (n = 3).

### 4. Temperature stability

When the temperature stability is evaluated, a mixture consisting of 5 µL of 806 µg/mL Protein 1 of the invention solution, 10 µL of 100 mM sodium acetate buffer (pH 6.0), and 35 µL of ultrapure water was maintained at each temperature (30 to 80°C) for 1 hour, and then water-cooled. After the water-cooling, the mixture was diluted to a Protein 1 concentration of 40.3 µg/mL using Dilution buffer A, and the resulting solution was used as a sample for each temperature. In addition, the 806 µg/mL Protein 1 solution was diluted to a concentration of 40.3 µg/mL using Dilution buffer A, and the resulting solution was used as a control.

To a mixture of 20 µL of 5 (w/v) % D-(+)-maltose, 20 µL of 100 mM sodium acetate buffer (pH 6.0), and 60 µL of ultrapure water, 10 µL of the sample for each temperature or control obtained by the dilution was added, and the resulting mixture was maintained at 40°C for 30 minutes. Then, 100 µL of 2 M Tris-HCl (pH 7.0) was added to terminate the reaction. To 100 µL of the reaction-terminated solution, 100 µL of Glucose C-II Test Wako was added, and the resulting mixture was maintained at 37°C for 20 minutes. Then, the absorbance of the mixture was measured at 492 nm, and the amount of glucose produced by the reaction was determined to measure the residual activity.

The residual activity was calculated as the ratio (%) of the activity of the sample for each temperature to the activity of the control. When the temperature stable range is defined as a range in which a residual activity of 90% or more is observed, the temperature stable range of this protein was up to 50°C (Fig. 5, white circles). The temperature stability was measured in triplicate (n = 3).

### Example 4: Saccharification test 1

### 1. Method

A reaction mixture was prepared by dissolving a maltohexaose and maltoheptaose high content syrup (G67-rich syrup), which was prepared by fractionation for this study, in ultrapure water to a final concentration of 30%, and adding a sodium acetate buffer (pH 5.0) at a final concentrations of 50 mM, CaCl2 at a final concentration of 3 mM, and NaN₃ at a final concentration of 0.02%. To the reaction solution, the culture supernatant of *Bacillus subtilis* prepared in Example 2 (Protein 2 of the invention) was added in a volume of 31.2, 62.4 or 125 µL/g-ds, and the reaction was allowed at 53°C for 72 hours. The reaction solution after the reaction was maintained in boiling water for 10 minutes to inactivate the enzyme, and the resulting sample was cooled and appropriately diluted, then desalted by addition of Amberlite MB4, and filtered through a 0.45 µm filter. The obtained reaction product was used for the subsequent analysis. In addition, as a control, the culture supernatant of *Bacillus subtilis* described in Patent document 8 (control enzyme: α-1,6-glucosyltransferase derived from *Thermoanaerobacter siderophilus*) was used, and the same operation as described above was performed with this enzyme. The culture supernatants used as Protein 2 of the invention and the control enzyme were obtained by culture performed under the same culture conditions.

The sugar composition was analyzed by HPLC of the reaction product. The analysis conditions were as follows: column, MCI GELCK02AS (Mitsubishi Chemical); eluent, ultrapure water; flow rate, 0.7 mL/min; column temperature, 80°C; and detector, differential refractive index detector. The ratio of each sugar in the sugar composition (%) of the reaction product was calculated as the area ratio (%) of the peak corresponding to the sugar to the total area of the peaks detected by HPLC, which was taken as 100. Since it had been revealed that a retention time of 30 minutes corresponds to DP30, the total of the areas of retention times shorter than 30 minutes was calculated as the area of DP31 or higher.

In order to confirm the amount of α-1,6 linkage in the reaction product, the reaction product obtained with Protein 2 of the invention was subjected to a dextranase treatment. For the dextranase treatment, 20 µL of Dextranase L "Amano" diluted 200 times in a 200 mM sodium acetate buffer (pH 5.0) was added to 0.5 mL of the sample containing 1% of solid content, and the mixture was maintained at 53°C for 24 hours. After the dextranase treatment, sugar composition analysis was performed. The sugar composition of the reaction product obtained with Protein 2 of the invention was compared with the sugar composition obtained after the dextranase treatment, and the increase in DP1 to 3 observed after the dextranase treatment was calculated ([Increase in DP1 to 3 (%)] = [Sugar content ratio of DP1 to 3 after dextranase treatment (%)] - [Sugar content ratio of DP1 to 3 before dextranase treatment (%)]) and the calculated increase in DP1 to 3 was used as index of the amount of α-1,6 linkage in the reaction product. The sugar composition of DP1 to 3 was analyzed under the following conditions: column, MCI GEL CK04S (Mitsubishi Chemical); eluent, ultrapure water; flow rate, 0.4 ml/minute; column temperature, 70°C; and detector, differential refractive index detector.

### 2. Results

Fig. 6 shows the chromatogram obtained by HPLC analysis of the G67-rich syrup. Figs. 7A and 7B show the chromatograms obtained by the HPLC analysis of the reaction products obtained with Protein 2 of the invention and the dextranase-treatment product thereof. Table 5 shows the sugar compositions of the G67-rich syrup, the reaction product obtained with Protein 2 of the invention, and the reaction product obtained with the control enzyme, as well as the increases (%) in DP1 to 3 after the dextranase treatment.

**[Table 5]**

| | | G67-Rich syrup | Reaction product 5-1 | Reaction product 5-2 | Reaction product 5-3 | Reaction product C-1 | Reaction product C-2 | Reaction product C-3 |
|---|---|---|---|---|---|---|---|---|
| Enzyme used | | - | Protein 2 of the invention | | | Control enzyme | | |
| Addition amount of enzyme (µL/g-ds) | | - | 31.2 | 62.4 | 125 | 31.2 | 62.4 | 125 |
| | | | | | | | | |
| Sugar composition of reaction product (%) | DP31 or higher | 0.4 | 0.5 | 0.8 | 0.8 | 3.7 | 2.4 | 1.7 |
| | DP20-30 | 0.0 | 0.6 | 1.8 | 4.3 | 0.1 | 0.0 | 1.9 |
| | DP10-20 | 2.1 | 40.2 | 42.7 | 41.4 | 27.6 | 35.2 | 41.7 |
| | DP3-9 | 97.2 | 53.0 | 47.9 | 45.7 | 64.8 | 56.6 | 47.6 |
| | DP1-2 | 0.3 | 5.7 | 6.7 | 7.7 | 3.8 | 5.8 | 7.1 |
| | | | | | | | | |
| Increase in DP1 to 3 (%) after dextranase treatment (%) | | - | 34.7 | 37.9 | 41.1 | 24.0 | 31.4 | 33.3 |

The starting material G67-rich syrup contained about 37% and 35% of DP6 and 7 carbohydrates, respectively, and about 97% of DP3 to 9 carbohydrates (Fig. 6, Table 5).

The contents of DP10 to 20 in the reaction products were significantly increased compared with the starting material. The contents of DP10 to 20 increased from 2.1% in the starting material to higher than 40% in all of the reaction products 5-1 to 5-3 obtained with Protein 2 of the invention, and increased to higher than 40% only in C-3 among the reaction products C-1 to C-3 obtained with the control enzyme (Fig. 7A, Table 5).

Both Protein 2 of the invention and the control enzyme showed a tendency that a higher addition amount of enzyme provides a larger increase in the percentage of DP1 to 2 content in the reaction product (Fig. 7A, Table 5).

Furthermore, Protein 2 of the invention showed a tendency that a higher addition amount of the enzyme provides a larger increase in the percentage of DP20 to 30 content in the reaction product (Fig. 7A, Table 5).

On the other hand, the content ratios of DP3 to 9 in both the reaction products obtained with the protein 2 and the control enzyme significantly decreased compared with the starting material. There was observed a tendency that a larger addition amount of the enzyme provides a lower content of DP3 to 9 in the reaction products. The content ratio of DP3 to 9 was 97.2% in the starting material, but decreased to 45.7% in the reaction product 5-3, in which the ratio was the lowest (Fig. 7A, Table 5).

As for the increase in DP1 to 3 in the reaction products treated with dextranase, the increases of DP1 to DP3 in all the reaction products 5-1 to 5-3 obtained with Protein 2 of the invention and the reaction products C-2 and C-3 obtained with the control (except for C-1) were higher than 30% (Fig. 7B, Table 5). Since the increase in DP1 to 3 was caused by the hydrolysis of the α-1,6 linkages by dextranase, it was revealed that the increased DP10 to 30 in the reaction products observed in this example were generated by the α-1,6 transfer activity of Protein 2 of the invention.

As shown in the results mentioned above, with Protein 2 of the invention, the percentage of DP10 or higher in the reaction product exceeded 40% even in the reaction product 5-1 obtained with the lowest enzyme addition amount (enzyme addition amount, 31.2 µL/g-ds). On the other hand, with the control enzyme, the ratio of DP10 or higher in the reaction product exceeded 40% only in the reaction product C-3, which was obtained with the highest enzyme addition amount (enzyme addition amount: 125 µL/g-ds). As for comparison of the enzyme addition amounts, it was demonstrated that Protein 2 of the invention could produce the same level of a-1,6-glucan compared with the control enzyme with an addition amount corresponding to one-fourth of that of the control enzyme.

In other words, it was demonstrated that when the protein is used as a culture supernatant of *Bacillus subtilis,* the protein of the invention derived from *Tepidibacillus decaturensis* can more efficiently produce α-1,6-glucans compared with the control enzyme derived from *Thermoanaerobacter siderophilus.*

### Example 5: Saccharification test 2

### 1. Method

To a starch partial degradation product (B x 27.1, also called liquefying liquid), which was prepared for the present study, an MES-NaOH buffer, pH 6.0 was added at a final concentration of 50 mM, NaN₃ was added at a final concentration of 0.02%, and the resulting mixture was maintained at 53°C. There were added 31.2 µL/g-ds or 125 µL/g-ds of Protein 2 of the invention, 200 U/g-ds of GODO-FIA (Godo Shusei, FIA), and 0.2 mg/g-ds of pullulanase "Amano" 3 (Amano Enzyme, PUL3). In addition, 0.01 mg/g-ds of Kleistase L-1 (Amano Enzyme, L-1) was added at the start of the reaction and after 24 hours of the reaction. The reaction mixture was sampled over time, and subjected to a dextranase treatment and sugar composition analysis. To the reaction mixture obtained after 72 hours of the reaction, which was adjusted to pH 6.0, 0.2 mg/g-ds of Kleistase L-1 was added, and the resulting mixture was maintained at 80°C to perform an iodine scavenging reaction. Color development with iodine was confirmed by adding 40 µL of 10 mM iodine solution to 1 mL of the reaction mixture diluted to B x 5. In both systems, the color development with iodine disappeared after 1 hour of the reaction, and therefore the reaction mixture was adjusted to pH 4.0, and maintained in boiling water for 10 minutes to inactivate the enzyme. The reaction mixture after the iodine scavenging was desalted with Amberlite MB4, filtered through a 0.45 µm filter, and used for the subsequent analysis. The analysis of the sugar composition of the reaction product was carried out in the same manner as in Example 4, and the conditions of the dextranase treatment were the same as those mentioned in Example 4.

### 2. Results

**[Table 6]**

| | | Starch partial degradation product liquefying liquid | Reaction product 6-1 | Reaction product 6-2 |
|---|---|---|---|---|
| Enzyme used | | - | Protein 2 of the invention | |
| Addition amount of enzyme of the invention (µL/g-ds) | | - | 31.2 | 125 |
| Addition of L1 [mg/g-ds] at 0 h | | - | 0.01 | 0.01 |
| Addition of L1 [mg/g-ds] at 24 h | | - | 0.01 | 0.01 |
| FIA [U/g-ds] | | - | 200 | 200 |
| PUL3 [mg/g-ds] | | - | 0.2 | 0.2 |
| Iodine-scavenged L-1 [mg/g-ds] | | - | 0.2 | 0.2 |
| | | | | |
| Sugar composition of reaction product(%) | DP31 or higher | 71.7 | 1.3 | 2.9 |
| | DP20-30 | 7.4 | 11.7 | 20.8 |
| | DP10-20 | 11.8 | 51.1 | 42.3 |
| | DP3-9 | 8.7 | 31.5 | 28.0 |
| | DP1-2 | 0.4 | 4.4 | 6.0 |
| | | | | |
| Increase in DP1 to 3 (%) after dextranase treatment | | - | 45.1 | 47.8 |

The liquefying liquid as the starting material contained 71.7% of DP31 or higher and 11.8% of DP10 to 20 (Table 6, Fig. 8).

In the reaction product obtained with Protein 2 of the invention, DP31 or higher was markedly decreased compared with that obtained with the liquefying liquid of the starting material (71.7%) after 72 hours of the reaction (after iodine scavenging), and the content thereof was 1.3% in the reaction product 6-1, and 2.9% in the reaction product 6-2. On the other hand, the DP 10 to 20 in the reaction products significantly increased compared with the liquefying liquid as the starting material (11.8%), and the content thereof was 51.1% in the reaction product 6-1, and 42.3% in the reaction product 6-2 (Table 6, Fig. 9A).

After the iodine scavenging, the increase in DP1 to 3 observed after the dextranase treatment was 45.1% in the reaction product 6-1, and 47.8% in the reaction product 6-2 (Table 6, Fig. 9B), which were larger than those observed when the substrate was G67-rich syrup (Table 5, 34.7 to 41.1%). In addition, when the increase in DP1 to DP3 was examined over time, it was higher than 30% in both the reaction products 6-1 and 6-2 after 24 hours of the reaction, and increased to about 45% after 48 hours of the reaction (Fig. 10). It was found that the α-1,6-transfer reaction reached near a plateau after 48 hours of the reaction (Fig. 10).

These results indicate that the protein of the invention can efficiently produce α-1,6-glucans by using multiple hydrolytic enzymes in combination, even when a starch partial degradation product (liquefying liquid) is used as a substrate.

### Industrial applicability

The present invention is useful in a variety of fields such as food and medicine where α-1,6-glucans can be used.

### Sequence Listing Free Text

SEQ ID NOS: 1 and 3, Amino acid sequences of a protein derived from *Tepidibacillus decaturensis*
SEQ ID NOS: 2 and 4, Nucleotide sequences encoding the amino acid sequences of SEQ ID NOS: 1 and 3
SEQ ID NOS: 5 to 11, Nucleotide sequences of primers

## Claims

1. A protein having an activity for catalyzing an a-1,6-glucosyl transfer reaction, which is any of the proteins (a) to (c) mentioned below:
(a) a protein consisting of the amino acid sequence of SEQ ID NO: 3:
(b) a protein consisting of an amino acid sequence having an amino acid sequence identity of 90% or higher to the amino acid sequence of SEQ ID NO: 3; and
(c) a protein consisting of an amino acid sequence in which one or several amino acid(s) have been substituted, inserted, deleted and/or added in the amino acid sequence of SEQ ID NO: 3.

2. A protein derived from *Tepidibacillus deca turensis* and having the following properties:
(1) the protein has an activity for catalyzing an a-1,6-glucosyl transfer reaction;
(2) the molecular weight measured by SDS-PAGE is from 95,000 to 105,000;
(3) the optimum pH is 3.9 to 4.6;
(4) the stable pH range is 4.2 to 9.0;
(5) the optimum temperature is 50 to 55°C; and
(6) the temperature stability is maintained at 50°C or lower.

3. A culture supernatant of a transformed cell into which any of the polynucleotides (a) to (c) mentioned below has been introduced:
(a) a polynucleotide consisting of the nucleotide sequence of SEQ ID NO: 4;
(b) a polynucleotide that hybridizes with a complementary strand of the polynucleotide consisting of the nucleotide sequence of SEQ ID NO: 4 under stringent conditions; and
(c) a polynucleotide having a sequence identity of 90% or higher to the polynucleotide consisting of the nucleotide sequence of SEQ ID NO: 4

4. An enzyme preparation for use in production of an α-1,6-glucan from an oligosaccharide and/or polysaccharide having an α-1,4-glucosidic linkage and/or an α-1,6-glucosidic linkage, which contains the protein according to claim 1 or 2 and/or the culture supernatant according to claim 3.

5. The enzyme preparation according to claim 4, wherein the oligosaccharide and/or polysaccharide having an α-1,4-glucosidic linkage and/or an α-1,6-glucosidic linkage is a starch partial degradation product.

6. The enzyme preparation according to claim 4 or 5, wherein the α-1,6-glucan is an isomaltooligosaccharide and/or isomaltomegalosaccharide having a degree of polymerization of from 2 to 30.

7. A composition containing any of the proteins (a) to (c) mentioned below and having an activity for catalyzing an a-1,6-glucosyl transfer reaction:
(a) a protein consisting of the amino acid sequence of SEQ ID NO: 3;
(b) a protein consisting of an amino acid sequence having an amino acid sequence identity of 90% or higher to the amino acid sequence of SEQ ID NO: 3; and
(c) a protein consisting of an amino acid sequence in which one or several amino acid(s) have been substituted, inserted, deleted and/or added in the amino acid sequence of SEQ ID NO: 3.

8. The composition according to claim 7, which is for use in production of an α-1,6-glucan from an oligosaccharide and/or polysaccharide having an α-1,4-glucosidic linkage and/or an α-1,6-glucosidic linkage.

9. A method for producing an α-1,6-glucan, which comprises the reaction step of allowing the enzyme preparation according to any one of claims 4 to 6 or the composition according to claim 7 or 8 to act on an oligosaccharide and/or polysaccharide having an α-1,4-glucosidic linkage and/or an α-1,6-glucosidic linkage to obtain an α-1,6-glucan.

10. The production method according to claim 9, which comprises the following step to be performed prior to the aforementioned reaction step:
the step of hydrolyzing starch to obtain an oligosaccharide and/or polysaccharide having an α-1,4-glucosidic linkage and/or an α-1,6-glucosidic linkage.

11. A method for producing a food, feed, feed for fish culture, cosmetic, or medicament, which comprises the step of performing the method according to claim 9 or 10 to obtain an α-1,6-glucan, and the step of obtaining a food, feed, feed for fish culture, cosmetic, or medicament by using the α-1,6-glucan obtained in the foregoing step.

12. A method for producing a glycoside, which comprises the step of allowing the enzyme preparation according to any one of claims 4 to 6 or the composition according to claim 7 or 8 to act on a sugar acceptor and a sugar donor.

13. The method for producing a glycoside according to claim 12, wherein the sugar donor is a maltooligosaccharide.

14. The method for producing a glycoside according to claim 12 or 13, wherein the sugar acceptor is a compound having an alcoholic hydroxyl group or a compound having a phenolic hydroxyl group.

15. A method for producing a food, feed, feed for fish culture, cosmetic, or medicament, which comprises the step of performing the method according to any one of claims 12 to 14 to obtain a glycoside, and the step of obtaining a food, feed, feed for fish culture, cosmetic, or medicament by using the glycoside obtained in the foregoing step.


SEQUENCE LISTING

<110>  Nihon Shokuhin Kako Co., Ltd.

<120>  A protein exhibiting a-1,6-transferase activity

<130>  F20678H-WO

<160>  11

<170>  PatentIn version 3.5

<210>  1
<211>  920
<212>  PRT
<213>  Tepidibacillus decaturensis

<400>  1

Met Asp Arg Ile Met Lys Asn Lys Tyr Trp Ile Phe Met Leu Thr Val
1               5                   10                  15

Ile Leu Ile Thr Ser Ile Leu Phe Ala Pro Leu Gln Ile Thr Gln Ala
            20                  25                  30

Ser Thr Asp Thr Leu Pro Thr Asp Gln Ser Val Thr Ser Ile Tyr Leu
        35                  40                  45

Asn Asn Trp Lys Asp Ala Val Asn Ile Trp Met Ala Ser Glu Leu Ala
    50                  55                  60

Gln Thr Asp Thr Gly Asn Tyr Gly Pro Arg Ile Gly Glu Met Arg Ile
65                  70                  75                  80

Asp Asp Asp Tyr Thr Val Asn Gln Ile His Asp Tyr Ser Ala Phe Phe
                85                  90                  95

Arg Asp Glu Thr Asn Ala Val Lys Tyr Thr Glu Pro His Asn Phe Ala
            100                 105                 110

Ser Glu Ala Tyr Tyr Asp Asp Gly Gly Ile Leu Gln Thr Lys Tyr Leu
        115                 120                 125

Asp Tyr Asn Gly Ser Asn Leu Pro Ile Val Val Glu Lys Asp Phe Ala
    130                 135                 140

Met Val Pro Asn Glu Asp Phe Met Ile Ala Lys Tyr Thr Phe Thr Asn
145                 150                 155                 160

Asn Asn Ala Thr Ser Ile Asn Phe Asn Leu Leu Glu Gln Ile His Val
                165                 170                 175

Asn Asn Val Thr Lys Gly Ser Thr Asn Gln Thr His His Gly Trp Tyr
        180                 185                 190

Asp Asn Thr Arg Asn Thr Leu Phe Val Asp Met Thr Ser Ser Gly Gln
    195                 200                 205

Tyr Tyr Val Ala Leu Gly Ala Phe Gln Ser Ala Asp Gly Tyr Gln Val
    210                 215                 220

Ala Asp Asp Thr Glu Ser Asn Leu Gly Ala Asn Asp Val Ser Ala Trp
225                 230                 235                 240

Tyr Thr Phe Asp Asn Asn Gly Thr Val Lys Asn Asn Ser Asp Glu Tyr
                245                 250                 255

Ala Val Asp Ile Ser Val Ala Phe Gln Asp Gln Val Thr Ile Pro Ala
        260                 265                 270

Ser Gly Ser Val Ser Val Ser Phe Val Ala Thr Val Gln Asp Ser Leu
    275                 280                 285

Thr Asn Ala Gln Asn Ser Val Asp Arg Ala Leu Ser Gln Lys Ala Asp
    290                 295                 300

Tyr Trp Phe Thr Gln Thr Ser Asn Thr Tyr Ser Ser Trp Leu Ser Gln
305                 310                 315                 320

Gly Lys Thr Val Asn Phe Ala Asp Gly Gly Ile Asn Lys Thr Tyr Thr
                325                 330                 335

Arg Ala Leu Ile Thr Ile Lys Asn Ala Thr Asn Pro Thr Tyr Gly Ala
        340                 345                 350

Thr Pro Ala Thr Thr Asn Pro Ile Ala Tyr Gly Tyr Lys Val Trp Ala
    355                 360                 365

Arg Asp Ser Ala Val Thr Ala Met Val Leu Asp Gln Ala Gly Phe Tyr
    370                 375                 380

Asp Glu Ala Glu Lys Tyr Trp Tyr Trp Leu Gln Asp Arg Gln Gln Ser
385                 390                 395                 400

Asp Gly Thr Phe Lys Thr Thr Phe Asp Tyr Trp Thr Asn Asn Tyr Val
                405                 410                 415

Ser Phe Val Glu Pro Glu His Asp Ser Ile Gly Ile Phe Leu Val Gly
        420                 425                 430

Ala Tyr Gln His Tyr Lys Leu Thr Gly Asp Thr Thr Phe Leu Asn Ser
        435                 440                 445

Ile Trp Thr Lys Tyr Lys Lys Ser Ala Asp Phe Ile Trp Ser Asn Leu
    450                 455                 460

Gly Ser Asp Pro Tyr Gly Phe Gly Glu Glu Asp Ala Ser Ile Trp Glu
465                 470                 475                 480

Glu Gln Ile Glu Tyr Asn Ala Phe Thr Gln Ala Leu Tyr Val Ala Gly
                485                 490                 495

Leu Asp Ala Ala Gln His Met Ala Arg Ala Lys Gly Leu Asn Ser Leu
            500                 505                 510

Ala Asp Asp Tyr Asn Gly Ala Ala Ser Glu Ile Arg Ser Asn Ile Gln
        515                 520                 525

Lys Asp Asp Thr Trp Ser Pro Ser Gly Leu Trp Asn Val Asn Asn Gly
    530                 535                 540

Tyr Tyr Asn Arg Ala Val Asn Thr Asn Gly Thr Ala Arg Thr Leu Val
545                 550                 555                 560

Asp Gly Ser Ser Asn Ala Leu Ile Val Tyr Gly Val Ile Asp Ala Asn
                565                 570                 575

Ser Ser Arg Ala Asn Ser His Val Asn Lys Ile Lys Thr Asn Leu Gln
            580                 585                 590

His Asp Asn Tyr Gly Ile Ala Arg Tyr Asp Gly Asp Asp Phe Tyr Tyr
        595                 600                 605

Thr Ser Pro Tyr Ser Pro Gly Gly Asn Glu Ala Leu Ser Asp Glu Pro
    610                 615                 620

Ser Trp Pro Gln Met Ser Ala Tyr Ala Ala Leu His His Leu Tyr Arg
625                 630                 635                 640

Gly Glu Lys Gln Thr Ala Leu Asn Tyr Leu Lys Trp Ile Val Ser Arg
                645                 650                 655

Thr Ala Val Gly Tyr Met Ala Gln Gly Glu Ala Val Ser Arg Ile Ser
            660                 665                 670

Leu Lys Pro Leu Pro Ser Thr Met Val Glu Pro Val Thr Gly Ala Trp
        675                 680                 685

Phe Val Ile Thr Ala Leu Val Tyr Glu Asp Gln Ala Asp Ile Arg Val
    690                 695                 700

Ile Pro Pro Gln Phe Asn Ala Gly Ala Tyr Lys Ser Ile Asn Val Thr
705                 710                 715                 720

Thr Thr Val Ala Asn Asp Leu Ala Gln Trp Asn Asn Val Pro Tyr Tyr
                725                 730                 735

His Asp Arg Leu Ser Asp Ser Asp Ser Gly Ser Asp Asp Thr Asp Ile
            740                 745                 750

Ala Lys Val Tyr Val Ser Asn Asp Ala Asn Asn Leu Tyr Val Arg Ile
        755                 760                 765

Lys Asn Ala Ser His His Leu Ser Gly Tyr Asn Thr Ala Pro Arg Phe
    770                 775                 780

Ala Met Thr Val Tyr Ala Glu Asp Phe Lys His Ser Thr Ala Glu Ser
785                 790                 795                 800

Leu Thr Thr Gly Leu Tyr Gly Gly Asn Leu Asp Arg Ser Met Gln Tyr
                805                 810                 815

Met Val Ser Arg Trp Ser Asp Ser Gly Asp Phe Ala Lys Phe His Val
            820                 825                 830

Ser Asn Gly Ser Trp Thr Phe Glu Lys His Leu Ser Gly Met Thr Ile
        835                 840                 845

Pro Gln Trp Asp Val Asn Thr Gly Asp Ile Glu Met Val Val Pro Leu
    850                 855                 860

Ser Glu Leu Ser Ser Thr Gly Ser Val Asn Thr Gly Asp Trp Ser Asn
865                 870                 875                 880

Leu Asn Ile Val Leu Val Arg Gln Asp Pro Thr Thr Leu Asn Trp Asn
                885                 890                 895

Glu Asp Asp Leu Ile Val Ile His Tyr Arg Val Met Thr Ser Gly Glu
            900                 905                 910

Gln Trp Tyr Tyr Gly Asn Val Glu
        915                 920

<210>  2

<211>  2763
<212>  DNA
<213>  Tepidibacillus decaturensis

<400>  2
atggatagaa tcatgaaaaa caaatactgg atctttatgc tgacagttat ccttatcaca      60

tcaatcctgt ttgccccgct gcaaatcaca caggcaagca cagatacact gccgacagat     120

caaagcgtta catctatcta ccttaacaac tggaaagatg cggtgaacat ctggatggct     180

tcagaattag cccaaacaga tacaggcaat tatggaccga gaattggcga aatgcgcatc     240

gatgatgatt acacagttaa ccagatccat gattacagcg catttttccg cgatgaaaca     300

aacgcagtga aatatacaga accgcataat tttgcttctg aagcctatta tgatgatggc     360

ggaattttac agacaaaata cctggattac aacggatcaa atcttccgat cgttgtggaa     420

aaagattttg caatggtgcc gaatgaagat tttatgatcg cgaaatacac atttacaaat     480

aacaacgcta catctatcaa ctttaatctg cttgaacaaa tccatgtgaa caatgtcaca     540

aaaggatcaa caaaccagac acatcatggc tggtacgata acacaagaaa cacactgttt     600

gtggatatga catcaagcgg ccaatattat gtcgcactgg gagcgtttca atcagccgat     660

ggctatcagg tcgcagatga tacagaatct aacctgggcg cgaatgatgt ctcagcttgg     720

tacacatttg ataacaacgg aacagttaaa aacaatagcg atgaatatgc ggtcgatatt     780

tctgttgctt ttcaagatca ggtcacaatc ccggcaagcg gctctgtgtc agtcagcttt     840

gttgccacag tgcaagatag cttaacaaac gcacagaata gcgttgatag agccctgtct     900

caaaaagcag attactggtt tacacagaca tcaaacacat attcttcatg gctgagccag     960

ggaaaaacag ttaactttgc ggatggcgga attaataaaa catatacacg cgcgcttatt    1020

acaatcaaaa acgctacaaa tccgacatat ggcgctacac cggccacaac aaatccgatt    1080

gcctatggat ataaagtgtg ggcaagagat tcagcagtga cagcgatggt ccttgatcaa    1140

gccggctttt acgatgaagc agaaaaatac tggtactggt tacaggatcg ccaacagagc    1200

gatggaacat ttaaaacaac atttgattac tggacaaaca attatgtctc ttttgttgaa    1260

ccggaacatg attcaattgg catctttctg gtcggagcgt atcaacatta taaactgaca    1320

ggcgatacaa catttcttaa cagcatctgg acaaaataca aaaaatctgc ggattttatc    1380

tggtcaaatc ttggaagcga tccgtatggc tttggagaag aagatgcttc tatctgggaa    1440

gaacaaatcg aatacaacgc atttacacag gcgctttatg ttgctggctt agatgcagcg    1500

caacatatgg ctagagccaa aggattaaac tcactggctg atgattataa tggcgctgcc    1560

tctgaaattc gctcaaacat ccagaaagat gatacatggt ctccgtcagg actgtggaac    1620

gtcaacaatg gctattataa tagagcggtt aacacaaatg gaacagctcg cacacttgtt    1680

gatggcagct ctaacgcctt aattgtgtat ggcgtcatcg atgcgaattc aagcagagct    1740

aactcacatg tgaacaaaat caaaacaaac ttacagcatg ataattatgg catcgctcgc 1800

tacgatggag atgattttta ctacacatca ccgtacagcc cgggcggaaa tgaagcgctg 1860

agcgatgaac cgtcttggcc gcaaatgagc gcttatgcag cgcttcatca tctgtacaga 1920

ggagaaaaac agacagccct gaactatctt aaatggattg tcagcagaac agcagttggc 1980

tatatggccc aaggagaagc agtttctcgc atctcattaa aaccgctgcc gtctacaatg 2040

gttgaaccgg tgacaggcgc gtggtttgtg attacagctc ttgtctatga agatcaagcg 2100

gatattagag tgatcccgcc gcagtttaat gcaggagcgt ataaaagcat caacgttaca 2160

acaacagtgg cgaatgatct ggctcagtgg aacaatgtcc cgtattatca tgatcgcctt 2220

agcgattctg attcaggcag cgatgataca gatattgcga aagtttacgt gtctaacgat 2280

gctaacaacc tttacgtgag aatcaaaaac gcttcacatc atttaagcgg ctataataca 2340

gcaccgagat ttgcaatgac agtctatgcc gaagatttta aacattctac agcagaatca 2400

cttacaacag gattatatgg cggaaacctt gatagatcaa tgcaatacat ggtgagccgc 2460

tggtctgatt caggagattt tgcaaaattt catgtcagca acggctcttg gacatttgaa 2520

aaacatctgt ctggaatgac aattccgcag tgggatgtca atacaggaga tatcgaaatg 2580

gtcgttccgc tttctgaatt atcttcaaca ggctcagtta acacaggaga ttggagcaac 2640

ctgaatattg tccttgttag acaagatccg acaacattaa actggaacga agatgatctg 2700

atcgttatcc attaccgcgt gatgacaagc ggcgaacagt ggtattatgg aaatgtggaa 2760

taa 2763

<210> 3
<211> 888
<212> PRT
<213> Tepidibacillus decaturensis

<400> 3

Ser Thr Asp Thr Leu Pro Thr Asp Gln Ser Val Thr Ser Ile Tyr Leu
1               5                   10                  15

Asn Asn Trp Lys Asp Ala Val Asn Ile Trp Met Ala Ser Glu Leu Ala
            20                  25                  30

Gln Thr Asp Thr Gly Asn Tyr Gly Pro Arg Ile Gly Glu Met Arg Ile
        35                  40                  45

Asp Asp Asp Tyr Thr Val Asn Gln Ile His Asp Tyr Ser Ala Phe Phe
    50                  55                  60

Arg Asp Glu Thr Asn Ala Val Lys Tyr Thr Glu Pro His Asn Phe Ala
65                  70                  75                  80

Ser Glu Ala Tyr Tyr Asp Asp Gly Gly Ile Leu Gln Thr Lys Tyr Leu
                85                  90                  95

Asp Tyr Asn Gly Ser Asn Leu Pro Ile Val Val Glu Lys Asp Phe Ala
            100                 105                 110

Met Val Pro Asn Glu Asp Phe Met Ile Ala Lys Tyr Thr Phe Thr Asn
        115                 120                 125

Asn Asn Ala Thr Ser Ile Asn Phe Asn Leu Leu Glu Gln Ile His Val
    130                 135                 140

Asn Asn Val Thr Lys Gly Ser Thr Asn Gln Thr His His Gly Trp Tyr
145                 150                 155                 160

Asp Asn Thr Arg Asn Thr Leu Phe Val Asp Met Thr Ser Ser Gly Gln
                165                 170                 175

Tyr Tyr Val Ala Leu Gly Ala Phe Gln Ser Ala Asp Gly Tyr Gln Val
            180                 185                 190

Ala Asp Asp Thr Glu Ser Asn Leu Gly Ala Asn Asp Val Ser Ala Trp
        195                 200                 205

Tyr Thr Phe Asp Asn Asn Gly Thr Val Lys Asn Asn Ser Asp Glu Tyr
    210                 215                 220

Ala Val Asp Ile Ser Val Ala Phe Gln Asp Gln Val Thr Ile Pro Ala
225                 230                 235                 240

Ser Gly Ser Val Ser Val Ser Phe Val Ala Thr Val Gln Asp Ser Leu
                245                 250                 255

Thr Asn Ala Gln Asn Ser Val Asp Arg Ala Leu Ser Gln Lys Ala Asp
            260                 265                 270

Tyr Trp Phe Thr Gln Thr Ser Asn Thr Tyr Ser Ser Trp Leu Ser Gln
        275                 280                 285

Gly Lys Thr Val Asn Phe Ala Asp Gly Gly Ile Asn Lys Thr Tyr Thr
    290                 295                 300

Arg Ala Leu Ile Thr Ile Lys Asn Ala Thr Asn Pro Thr Tyr Gly Ala
305                 310                 315                 320

Thr Pro Ala Thr Thr Asn Pro Ile Ala Tyr Gly Tyr Lys Val Trp Ala
                325                 330                 335

Arg Asp Ser Ala Val Thr Ala Met Val Leu Asp Gln Ala Gly Phe Tyr
            340                 345                 350

Asp Glu Ala Glu Lys Tyr Trp Tyr Trp Leu Gln Asp Arg Gln Gln Ser
        355                 360                 365

Asp Gly Thr Phe Lys Thr Thr Phe Asp Tyr Trp Thr Asn Asn Tyr Val
    370                 375                 380

Ser Phe Val Glu Pro Glu His Asp Ser Ile Gly Ile Phe Leu Val Gly
385                 390                 395                 400

Ala Tyr Gln His Tyr Lys Leu Thr Gly Asp Thr Thr Phe Leu Asn Ser
                405                 410                 415

Ile Trp Thr Lys Tyr Lys Lys Ser Ala Asp Phe Ile Trp Ser Asn Leu
            420                 425                 430

Gly Ser Asp Pro Tyr Gly Phe Gly Glu Glu Asp Ala Ser Ile Trp Glu
        435                 440                 445

Glu Gln Ile Glu Tyr Asn Ala Phe Thr Gln Ala Leu Tyr Val Ala Gly
    450                 455                 460

Leu Asp Ala Ala Gln His Met Ala Arg Ala Lys Gly Leu Asn Ser Leu
465                 470                 475                 480

Ala Asp Asp Tyr Asn Gly Ala Ala Ser Glu Ile Arg Ser Asn Ile Gln
                485                 490                 495

Lys Asp Asp Thr Trp Ser Pro Ser Gly Leu Trp Asn Val Asn Asn Gly
            500                 505                 510

Tyr Tyr Asn Arg Ala Val Asn Thr Asn Gly Thr Ala Arg Thr Leu Val
        515                 520                 525

Asp Gly Ser Ser Asn Ala Leu Ile Val Tyr Gly Val Ile Asp Ala Asn
    530                 535                 540

Ser Ser Arg Ala Asn Ser His Val Asn Lys Ile Lys Thr Asn Leu Gln
545                 550                 555                 560

His Asp Asn Tyr Gly Ile Ala Arg Tyr Asp Gly Asp Asp Phe Tyr Tyr
                565                 570                 575

Thr Ser Pro Tyr Ser Pro Gly Gly Asn Glu Ala Leu Ser Asp Glu Pro

            580                 585                 590

Ser Trp Pro Gln Met Ser Ala Tyr Ala Ala Leu His His Leu Tyr Arg
        595                 600                 605

Gly Glu Lys Gln Thr Ala Leu Asn Tyr Leu Lys Trp Ile Val Ser Arg
    610                 615                 620

Thr Ala Val Gly Tyr Met Ala Gln Gly Glu Ala Val Ser Arg Ile Ser
625                 630                 635                 640

Leu Lys Pro Leu Pro Ser Thr Met Val Glu Pro Val Thr Gly Ala Trp
                645                 650                 655

Phe Val Ile Thr Ala Leu Val Tyr Glu Asp Gln Ala Asp Ile Arg Val
            660                 665                 670

Ile Pro Pro Gln Phe Asn Ala Gly Ala Tyr Lys Ser Ile Asn Val Thr
        675                 680                 685

Thr Thr Val Ala Asn Asp Leu Ala Gln Trp Asn Asn Val Pro Tyr Tyr
    690                 695                 700

His Asp Arg Leu Ser Asp Ser Asp Ser Gly Ser Asp Asp Thr Asp Ile
705                 710                 715                 720

Ala Lys Val Tyr Val Ser Asn Asp Ala Asn Asn Leu Tyr Val Arg Ile
                725                 730                 735

Lys Asn Ala Ser His His Leu Ser Gly Tyr Asn Thr Ala Pro Arg Phe
            740                 745                 750

Ala Met Thr Val Tyr Ala Glu Asp Phe Lys His Ser Thr Ala Glu Ser
        755                 760                 765

Leu Thr Thr Gly Leu Tyr Gly Gly Asn Leu Asp Arg Ser Met Gln Tyr
    770                 775                 780

Met Val Ser Arg Trp Ser Asp Ser Gly Asp Phe Ala Lys Phe His Val
785                 790                 795                 800

Ser Asn Gly Ser Trp Thr Phe Glu Lys His Leu Ser Gly Met Thr Ile
                805                 810                 815

Pro Gln Trp Asp Val Asn Thr Gly Asp Ile Glu Met Val Val Pro Leu
            820                 825                 830

Ser Glu Leu Ser Ser Thr Gly Ser Val Asn Thr Gly Asp Trp Ser Asn
        835                 840                 845

Leu Asn Ile Val Leu Val Arg Gln Asp Pro Thr Thr Leu Asn Trp Asn
    850                 855                 860

Glu Asp Asp Leu Ile Val Ile His Tyr Arg Val Met Thr Ser Gly Glu
865                 870                 875                 880

Gln Trp Tyr Tyr Gly Asn Val Glu
                885

<210>  4
<211>  2667
<212>  DNA
<213>  Tepidibacillus decaturensis

<400>  4
agcacagata cactgccgac agatcaaagc gttacatcta tctaccttaa caactggaaa      60

gatgcggtga acatctggat ggcttcagaa ttagcccaaa cagatacagg caattatgga     120

ccgagaattg gcgaaatgcg catcgatgat gattacacag ttaaccagat ccatgattac     180

agcgcatttt tccgcgatga aacaaacgca gtgaaatata cagaaccgca taattttgct     240

tctgaagcct attatgatga tggcggaatt ttacagacaa aatacctgga ttacaacgga     300

tcaaatcttc cgatcgttgt ggaaaaagat tttgcaatgg tgccgaatga agattttatg     360

atcgcgaaat acacatttac aaataacaac gctacatcta tcaactttaa tctgcttgaa     420

caaatccatg tgaacaatgt cacaaaagga tcaacaaacc agacacatca tggctggtac     480

gataacacaa gaaacacact gtttgtggat atgacatcaa gcggccaata ttatgtcgca     540

ctgggagcgt ttcaatcagc cgatggctat caggtcgcag atgatacaga atctaacctg     600

ggcgcgaatg atgtctcagc ttggtacaca tttgataaca acggaacagt taaaaacaat     660

agcgatgaat atgcggtcga tatttctgtt gcttttcaag atcaggtcac aatcccggca     720

agcggctctg tgtcagtcag ctttgttgcc acagtgcaag atagcttaac aaacgcacag     780

aatagcgttg atagagccct gtctcaaaaa gcagattact ggtttacaca gacatcaaac     840

acatattctt catggctgag ccagggaaaa acagttaact ttgcggatgg cggaattaat     900

aaaacatata cacgcgcgct tattacaatc aaaaacgcta caaatccgac atatggcgct     960

acaccggcca caacaaatcc gattgcctat ggatataaag tgtgggcaag agattcagca    1020

gtgacagcga tggtccttga tcaagccggc ttttacgatg aagcagaaaa atactggtac    1080

tggttacagg atcgccaaca gagcgatgga acatttaaaa caacatttga ttactggaca    1140

aacaattatg tctcttttgt tgaaccggaa catgattcaa ttggcatctt tctggtcgga    1200

gcgtatcaac attataaact gacaggcgat acaacatttc ttaacagcat ctggacaaaa    1260

tacaaaaaat ctgcggattt tatctggtca aatcttggaa gcgatccgta tggctttgga 1320

gaagaagatg cttctatctg ggaagaacaa atcgaataca acgcatttac acaggcgctt 1380

tatgttgctg gcttagatgc agcgcaacat atggctagag ccaaaggatt aaactcactg 1440

gctgatgatt ataatggcgc tgcctctgaa attcgctcaa acatccagaa agatgataca 1500

tggtctccgt caggactgtg gaacgtcaac aatggctatt ataatagagc ggttaacaca 1560

aatggaacag ctcgcacact tgttgatggc agctctaacg ccttaattgt gtatggcgtc 1620

atcgatgcga attcaagcag agctaactca catgtgaaca aaatcaaaac aaacttacag 1680

catgataatt atggcatcgc tcgctacgat ggagatgatt tttactacac atcaccgtac 1740

agcccgggcg gaaatgaagc gctgagcgat gaaccgtctt ggccgcaaat gagcgcttat 1800

gcagcgcttc atcatctgta cagaggagaa aaacagacag ccctgaacta tcttaaatgg 1860

attgtcagca gaacagcagt tggctatatg gcccaaggag aagcagtttc tcgcatctca 1920

ttaaaaccgc tgccgtctac aatggttgaa ccggtgacag gcgcgtggtt tgtgattaca 1980

gctcttgtct atgaagatca agcggatatt agagtgatcc cgccgcagtt taatgcagga 2040

gcgtataaaa gcatcaacgt tacaacaaca gtggcgaatg atctggctca gtggaacaat 2100

gtcccgtatt atcatgatcg ccttagcgat tctgattcag gcagcgatga tacagatatt 2160

gcgaaagttt acgtgtctaa cgatgctaac aacctttacg tgagaatcaa aaacgcttca 2220

catcatttaa gcggctataa tacagcaccg agatttgcaa tgacagtcta tgccgaagat 2280

tttaaacatt ctacagcaga atcacttaca acaggattat atggcggaaa ccttgataga 2340

tcaatgcaat acatggtgag ccgctggtct gattcaggag attttgcaaa atttcatgtc 2400

agcaacggct cttggacatt tgaaaaacat ctgtctggaa tgacaattcc gcagtgggat 2460

gtcaatacag gagatatcga aatggtcgtt ccgctttctg aattatcttc aacaggctca 2520

gttaacacag gagattggag caacctgaat attgtccttg ttagacaaga tccgacaaca 2580

ttaaactgga acgaagatga tctgatcgtt atccattacc gcgtgatgac aagcggcgaa 2640

cagtggtatt atggaaatgt ggaataa 2667

<210> 5
<211> 30
<212> DNA
<213> Artificial sequence

<220>
<223> primer

<400> 5
actgctcttg gatccagcac agatacactg 30

<210> 6

<211>  39
<212>  DNA
<213>  Artificial sequence

<220>
<223>  primer

<400>  6
atggtgatgg tggtgttcca catttccata ataccactg                              39


<210>  7
<211>  42
<212>  DNA
<213>  Artificial sequence

<220>
<223>  primer

<400>  7
caccaccatc accatcattg agtcgacctg cagatctcta ga                          42


<210>  8
<211>  18
<212>  DNA
<213>  Artificial sequence

<220>
<223>  primer

<400>  8
ggatccaaga gcagtggc                                                     18


<210>  9
<211>  33
<212>  DNA
<213>  Artificial sequence

<220>
<223>  primer

<400>  9
gatctgcagg tcgacttatt ccacatttcc ata                                    33


<210>  10
<211>  20
<212>  DNA
<213>  Artificial sequence

<220>
<223>  primer

<400>  10
gtcgacctgc agatctctag                                                   20


<210>  11
<211>  17
<212>  DNA
<213>  Artificial sequence

<220>
<223>  primer

<400>  11
ggatccaaga gcagtgg                                                    17